# EUROPEAN PATENT APPLICATION

(11) **EP 1 324 042 A2**
(43) Date of publication of application: **02.07.2003**
(21) Application number: 02447277.1
(22) Date of filing: 24.12.2002
(51) Int. Cl.: G01N 33/543, C12Q 1/68, B01J 19/00

(54) **Detection and/or quantication method of a target molecule by a binding with a capture molecule fixed on the surface of a disc**

(30) Priority: 27.12.2001 US 35822
(71) Applicant: Remacle, José, 5020 Malonne (BE)
(72) Inventor: Remacle, José, 5020 Malonne (BE); Alexandre, Isabelle, 5340 Haltinne (BE); Houbion, Yves, 5150 Floreffe (BE)
(74) Representative: Van Malderen, Joelle

(57) **Abstract**

The present invention is related to a method for the detection and/or the quantification of a target molecule by its binding with a corresponding capture molecule fixed on the side of a disc opposite to the side comprising registered data.

The present invention is also related to a disc having fixed upon its surface said capture molecules, to its preparation process, and to a diagnostic and/or reading device of said disc or comprising said disc.

SEQUENCE LISTING

<110> Remacle, José

<120> DETECTION AND/OR QUANTIFICATION METHOD OF A TARGET MOLECULE BY A BINDING
WITH A CAPTURE MOLECULE FIXED ON THE SURFACE OF A DISC

<130> P.FNDP.03B/EP

<160> 4

<170> PatentIn version 3.1

<210> 1
<211> 22
<212> DNA
<213> Artificial Sequence

<220>
<223> consensus primer for fem A : Apcons31
<400> 1
taayaaartc accaacatay tc 22

<210> 2
<211> 22
<212> DNA
<213> Artificial Sequence

<220>
<223> consensus primer for fem A : Apcons32
<220>
<221> misc_feature
<222> (5)..(5)
<223> Any nucleotide

<400> 2
tymgntcatt tatggaagat ac 22

<210>  3

<211>  21

<212>  DNA

<213>  Artificial Sequence

<220>

<223>  primer for mec A : Apmec01

<400>  3
tctggaactt gttgagcaga g                                                21

<210>  4

<211>  22

<212>  DNA

<213>  Artificial Sequence

<220>

<223>  primer for mec A : Apmec02

<400>  4
ggctatcgtt gtcacaatcg tt                                               22

## Description

### Field of the invention

The present invention is related to a detection and/or quantification method of a target molecule by its binding with a capture molecule fixed on the surface of a disc.

The present invention is also related to a disc having fixed upon its surface a non-cleavable capture molecule, to its preparation process, and to a diagnostic and/or reading device of said disc or comprising said disc.

### Background of the invention

The international patent application WO99/35499 describes the detection and/ or quantification method of one or more target molecules present in a sample, by allowing a binding between said target molecule an a capture molecule bound upon the surface of a solid support, being a disc comprising registered data; said binding resulting in a signal. Said method allows a detection and/or quantification of said signal with the proviso that said signal is not obtained from a cleavage of said capture molecule and the signal for the detection, identification and/ or quantification of the target molecules is present and read on an area of the disc different from an area wherein the registered data are present.

The document WO98/01533 describes a cleavable signal element (or "cleavable capture molecule") comprising a cleavable spacer having a substrate-attaching end (which can be a compact-disc), a signal-responsive end (which can be linked to a metallic beads, especially gold beads), and a first side member adapted to bind a first site on a chosen analyte and a second side member adapted to bind a second site of said chosen analyte. The signal is measured when the analyte is fixed upon the first side member and the second side member. Thereafter, the spacer is cleaved and the fixation of the analyte allows the detection of a positive signal.

However, this complex and expensive detection method and device is submitted to various false positives or false negatives in the detection of various complex analytes, which could develop various interactions with said cleavable signal elements.

### Summary of the invention

The present invention is related to a method for the detection and/or the quantification of a target molecule as described in the claims.

The present invention is also related to a disc having fixed upon its surface a non-cleavable capture molecule as described in the claims, and which can be used in the detection and/or quantification method according to the invention.

Another aspect of the present invention is related to a preparation process and apparatus for obtaining said disc, a diagnostic kit comprising said disc, a diagnostic and reading device comprising said disc or a diagnostic and reading device which allows the reading and the analysis of the data present upon the disc according to the invention. The method and means according to the invention are particularly well suited for analysis of multiple possible targets present in a sample using microarrays and for multiple samples analysis upon a same disc.

### Definitions

The definition and characteristics of the terms "compact-disc "disc platform", "mini CD", "data that can be read by a CD-reading device including possibly registered data", "target" and " capture molecules" are already described in the document WO99/35499 incorporated herein by reference. Compact-disc such as CD, or DVD being used according to this invention for idenitification of biological molecules are refered as Bio-CD. "Cleavable capture molecules fixed upon the surface of a disc" correspond to the ones described in the patent application WO98/01533.

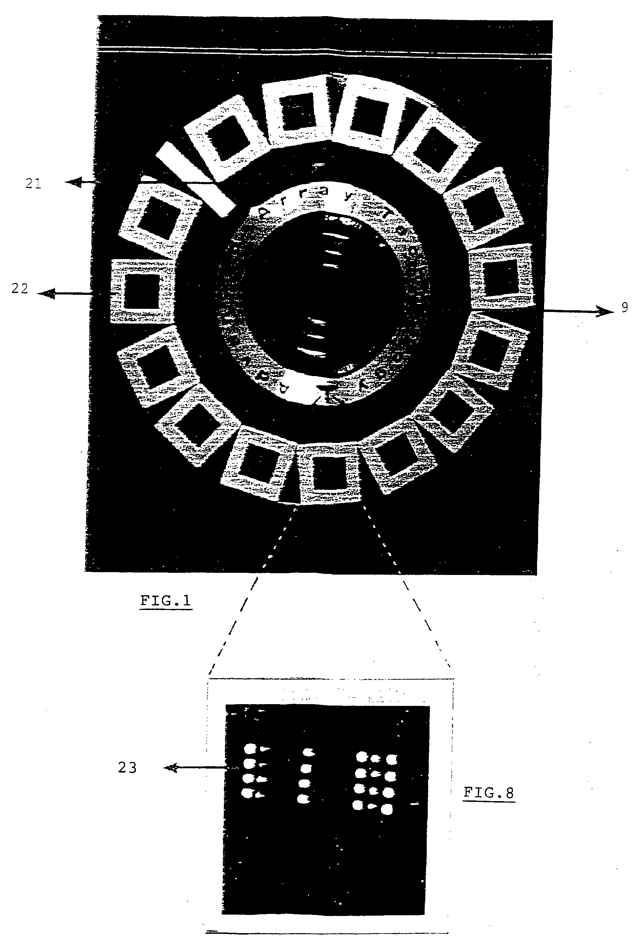

By the term "disc" is meant a flat solid support(s) (usually in the form of a disc) which comprises a hole that allows its rotation according to an axis (A) which is located in the center of said hole), made in a rigid material comprising usually one or more polymer layers (like a polyacrylic layer) and which can be covered by one or more metal layers (like gold or aluminum thin layers) so as to allow penetration and reflection of a light beam, preferably a laser beam 7, which is used for the detection and the reading of registered data present in (circular or spiral) tracks upon said disc (see Fig. 1).

The configuration of said polymeric and metallic layers is prepared in order to allow the penetration and the reflection of the laser beam upon selected layers. For instance, the disc may comprise a superior layer that allows the penetration of the laser beam, which will be reflected by a second lower metallic layer or the other way around.

The definition of a "disc" includes any solid support such as a Compact Disc (CD) or a "DVD" which comprises data than can be read by a CD-reading device (by penetration and reflection of a laser beam).

It is meant by "data than can be read by a CD-reading device", possibly registered data (i.e. about the characteristics of capture molecules upon specific areas of said solid support) or data used for the treatment of a signal which is the result of a binding between a target and a capture molecules.

Registered data are data physically inserted onto the disc [CD] preferably as pits and lands and which are read by a succession of 0 or 1 signals (See description in "The complete recordable-CD guide, L. Purcell, D. Martin, SYBEX, 1997, San Francisco, Paris, Dusseldorf, Soest"). In a classical CD reader the transition between the pits and the lands is indicated by binary 1. This transition is performed by a change in the reflexion of the laser bean. CDs belong to the family data storage which use laser bean to detect impressions in the surface of the reflective disc. The data store as pits or lands on CD exist in a continuous spiral track which has a physical support like in the CD-R or not. The sequence of the 0 or 1 corresponds to data bytes which are then converted into information like words, numbers, musics, softwares, data, ... in a similar way as the bytes of the computer information process. There are called numeric information.

Preferably, such readable registered data in the disc will be converted into one or several of the following information: data corresponding to the information necessary for the localisation and identification of the various arrays and capture molecules present on the CD as well as results from a positive binding of said target molecules upon capture molecules, the quantification process, data necessary for the computer to recognize the CD pattern and to identify the CD, data or to obtain a regular speed of the CD and possible recordable area. One or more sections of a disc such as a compact-disc are dedicated to data processing by standard read/write digital technology (CD spiral tracks). Specific data for processing and including information and instructions regarding the processing steps of the treatment of the biological sample (purification, washing, cutting, amplification, etc.) and analysis are recorded on the compact-disc surface using digital recording means.

Furthermore, read-only memory (ROM) on the disc comprises compact-disc information, instructions, experimental protocols, data analysis and statistical methods that can be accesses by a user operating the disc and recording of the binding location and result between the capture and target molecules.

Additionally, the disc may contain electronic circuitry, including microprocessors for a coordination of disc functions, and devices for communication with the disc manipulation and/or reading device or other devices. The disc optionally comprises detectors and sensors, or components of these devices and energy sources for various detection schemes (such as electric power supplies for electrochemical systems, electromagnetic radiation sources for spectroscopic systems), or materials, such as optically-transparent materials, that facilitate operation of and data generation using such detectors and sensors, actuators, communications and data handling devices, mediating communications between the disc and the player/reader device, using electromagnetic (laser, infrared, radiofrequency, microwave), electrical, or other means; circuitry designed for controlling procedures and processes on the disc, including systems diagnostics, assays protocols and analysis of assay data. These are preferably provided in the form of ASICs or ROM which are programmed only at the point-of-manufacture; FGPA's EPROM, flash memory (UV-erasable EPROM), or programmable IC arrays, or similar arrays programmable by the user through the platform manipulation device or other device. Also included in the components of the invention are CPU and microprocessor units and associated RAM operating with an assembler language or high-level language programmable through disc communications, and components for mediating communication with other devices, including facsimile/modem communications with remote display or data analysis systems.

A "disc platform" means one or more material(s) which is (are) turning along an axe (A') perpendical to the disc and which can be used for performing some or all the processes described herein (like, but not limited to data reading, data printing, washing step, incubation step with various solutions, illumination and light diffraction measure step, absorption or reflexion analysis.

The disc platform has usually the shape of a disc or contain at least a part having a disc shape or circular( spiral) shape tracks. It is composed either of one entity like the normal radio or recording CD or composed of several pieces, having or not the form of a disc, but which once reassociated show the form of a disc or at least contain a part having disc shape or circular(spiral) shape tracks. The disc platform can also comprised pieces which attach or are deposited in contact with the disc for performing the necessary steps for the analysis of the nucleotide targets such as the extraction, amplification, labelling and analysis.

A Mini-CD means a compact disc having a diameter size of 3-inch or smaller or about 3.8 cm and thickness of about 1 mm. The mini-CD can bear registered data on some locations of the CD or have a series of layers allowing data to be registered. The mini disc can have parts of the support located outside the tracks with are used in this invention for target analysis. These parts may be or circular shape or of another shape. One particular interesting mini-CD is the rectangular shape having the size of a credit card.

The biochemical terms, nucleic acids, oligonucleotides, nucleotide triphosphate homologous sequences and primer sequences are defined in the documents WO97/27317, WO00/72018 and EP-1136566 incorporated herein by reference.

### Brief description of the drawings

Figure 1 shows a Bio-CD containing arrays for making 15 sample analysis.

Figure 2 shows a mold containing 20 cavities which once in contact with the disc will provide 20 chambers of incubation for a sample to be analyzed.

Figure 3 shows the disposition of various types of chambers on the disc for making the steps necessary before a nucleotide analysis.

Figure 4 shows an automated incubation handling machine composed of a disc, a molded platform for making incubation chambers on the disc, a heating plate and a rotating device composed of two axes perpendical to each other.

Figures 5 to 8 show various types of Bio-CD reading devices.

Figure 9 shows an arrayer for the transfert of capture probes present in solution from multiwellplate onto the surface of Bio-CD.

Figure 10 shows the result of hybridization of a duplex PCR product made on staphyloccocus epidermidis methicillin resistant on a microarray on the surface of a disc. Each vertical rows are quadruplicates of the same capture probe. From left to right spotting controls, positive controls of hybridization, negative controls, S. aureus, S. epidermidis, S. haemoliticus, S. hominis, S. saprophyticus, mec A, S. consensus, positive controls.

Figure 11 gives the quantification of hybridizations of duplex PCR product from (2 million to 2 copies) of staphylococcus aureus methicillin resistant on the consensus capture probe of the staphylococcus microarray on the surface of a disc platform.

Figure 12 gives the result of hybridization of 9 duplex PCR products made on 9 different Staphyloccocus species methicillin resistant and 1 negative PCR on water.

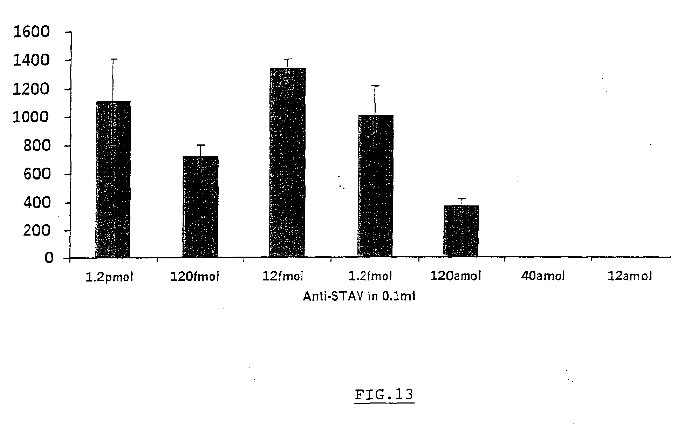

Figure 13 shows how two discs performing two different functions can be manufactured separetely and processed and then reassembled for the reading of the information.

Figure 14 gives the quantification of a concentration curve of antibody detection on protein chips spotted on the disc platform.

### Detailed description of the invention

A first aspect of the present invention is related to a method for the detection, identification and/or quantification of one or more target molecules present in a sample, preferably a biological sample comprising the steps of:
- allowing a binding between said target molecules and a capture molecule bound upon the surface of the solid support, being a disc comprising registered data (said disc being preferably a compact disc) said binding resulting in a signal, and
- allowing a detection and/or quantification of said signal.

The method according to the invention is also characterised by the following specific limitations:
- the signal is not obtained through cleavage of said capture molecule, the signal resulting from the detection, identification and/ or quantification of the target molecule is present and/ or read on one side of the disc, opposite to the side of the disc comprising registered data both area covering partially of totally the same locations of the disc.

A compact disc allows its rotation according to an axis, which is located in the centre of said hole and is made of rigid material, covered or not with dyes (for R-CD) and by one or more metal layers (such as gold or aluminium thin layers) and comprising usually one or more protective polymeric layers (like polyacrylic layers), the rigid material being transparent so as to allow penetration, reflection of a light beam preferably a laser beam, which is used for the detection and the reading of data or registered data present in circular or spiral tracks upon said disc. The laser can also be used for engraving the data in the Read and Write CD reader.

Therefore, as one side of the compact disc is made of transparent material for the support of registered data present in said circular or spiral tracks, the opposite side is made of a metallic layer recovered by a polymeric prtotective layer.

Thus, due to the specific reflective characteristics of said metallic layer, it was never proposed and never suggested in the state of the art to obtain the detection of a signal resulting from the binding of a target molecule on areas of the disc surface comprising said metallic layer. Indeed, due to the reflective characteristics of the metallic layer, it was proposed in the state of the art (WO 99/35499) to obtain a binding of capture molecules on locations of the disc which are different from locations comprising registered data, said locations comprising bounded capture molecules upon a transparent polymeric layer only and without the presence of the underline metallic layer

Contrary to the teaching of the state of the art, the inventors have discovered unexpectedly that it is possible to obtain, an efficient fixation of capture molecules upon the polymeric support present on the side of the disc made of the metallic layer recovered by a polymeric support.

Thereafter and unexpectedly, an efficient detection of the signal could be obtained (following the binding of the target and its corresponding capture molecule) by the formation of a precipitate upon the surface of the polymeric layer recovering said metallic layer.

Said precipitate could be either an opaque precipitate or a magnetic precipitate, more preferably a colloidal metallic reagent or compound such as a silver precipitate or a precipitate which induces a corrosion of the metallic layer of said disc. The signal resulting from said precipitate is advantageously read and/or recorded by a light beam (such as as laser beam) which is reflected by the metallic layer, but also diffracted when put in contact with the said precipitate, preferably with a colloidal metallic precipitate.

Therefore, the method according to the invention presents some specific advantages which are described in detail hereafter.

The method according to the invention allows an increasing of the surface dedicated to biological detection and an increasing of the surface dedicated to registered data. The overal area located on one side of the disc is available for numeric data registration while the overal area located on the other side of the disc is available for performing the handling of the biological sample or the different steps necessary for the detection of the target. The invention is particularly well suited for the multiple sample analysis of multiple possible targets being possibly present in the samples. The surface of the disc dedicated for biological analyis is divided in appropriate sections being at least 3 and more preferably at higher than 5, being for example 20, each section being used for performing an assay for one sample. Each of the section is separated from the neighbour by a physical or chemical boundary, which is detectable by the reading device and individualized so as to identify the analyzed sample. The handling of the liquid in each section of the disc is preferentially performed within incubation chambers obtained by adaptation of a physical layer onto the disc surface. The physical layer is preferentially removed for the reading in the reading device. The performance of the liquid handling on one side of the disc which do not contain the numeric information, allows the fixation of a physical layer in a strong way and the individual handling of each sample, being in different solutions or whashed or treated differently with more or less aggressive chemicals.

Furthermore, the method is well adapted for using standard CD with the appropriated layer for binding of capture probes. Also the automated production process of CD is available for the production of the disc according to the invention. One particular feature of the invention being the use of recordable CD being still able to be read and recorded even after having performed the overal steps necessary for the handling of the biological sample necessary for its detection on the same physical plateform.

Furthermore, the method and the disc according to the invention are extremely suitable for performing several steps either sequencely or simultaneously (a first step dedicated to the reading or recording of registered data upon one side of the disc and a second step dedicated to the biological reading and/or detection made upon the other side of the disc). Said steps could be performed by different media and means (the reading and the recording could be made by different reading and/or recording devices).

Said method also allows that the elements (tips and lands) present in the circular or spiral tract are not denaturated by means and media used in the biological detection step. Indeed, the signal resulting from the binding of a target molecule upon its capture molecule could also be obtained by a corrosion of one or more layers of the surface of the disc (polymeric and/or metallic layer). The formation of corroded spots upon said metallic layer could be also detected by the use of a light beam and a modification of the reflective and/or diffractive properties of said metallic layer.

Another aspect of the present invention concerns said disc or preferably said compact disc used in the method according to the invention.

One remarkable aspect of a compact disc according to the invention is the density of the microscopic array(s) of possibly registered data patterns embedded within the disc materials. It is an optical storage using a laser beam to detect impressions in the surface of the reflective disc. The ability to compress data to such a fine degree and read it back accurately gives the disc according to the invention one of its defining characteristics, the capability of storing huge amounts of data.

The compact disc according to the invention could be adapted for the penetration and reflection of various laser beams upon various polymeric or metallic layers.

The disc is in general of 1.2 mm thick and 4.72 inches in diameter, but smaller supports also exist and could be adapted for specific applications (such as binding between a capture and a target molecules into a Petri dish), and the thickness can be adapted according to the technical requirements of the capture molecule and the detection method of the invention used.

The disc can incorporate grooves to conduct the lecture by a laser beam. In said grooves are incorporated "registered" data that can be thereafter read, analyzed and advantageously Tran scripted into digital data or portions of the disc which may be engraved thereafter in order to add the data regarding the binding results obtained. Preferably, said registered data are in the form of binary information. These grooves may comprise fixed non-cleavable capture molecules.

Through its intensive and narrowly focused beam, the laser provides means for precise detection and registration of the passage of thousands of tiny impressions upon the rapidly spinning disc surface. Said detection process generates no friction since the detection is based on the measurement of phase shifts in reflected light. This technique allows the detection of considerable data compaction, since the carefully focused laser beam is able to respond at the speed of the light to extremely small variations in the disc surface.

Light derived from typically natural or artificial sources consists of photons that move in random wave patterns, even when they originate from light beams of the same frequency. Light beams of this sort are considered incoherent, meaning the waves travel in all directions. In comparison, the light associated with lasers is advantageously considered coherent.

A laser beam is created when a source of energy is introduced into what is called an *active medium.* A pair of mirrors positioned on each sides of an active medium are used to channel a portion of the radiation that strikes it. The active medium can consist of a gaseous mix (such as helium and neon) or ions within a crystalline matrix (such as found in the gallium-arsenide lasers typically used in compact-disc (CD) drives and recorders). The materials and the energy source used to stimulate the light determine the strength and intensity of the resulting beam. The lasers used within CD-equipment are usually of extremely low power.

The CD drive laser is directed at the spinning disc and the reflected light passes through a lens and strikes a photodiode (see Figs. 1 to 5). Data on the disc surface is encoded in the form of *pits* (indentations in the disc) and *lands* (the surface of the disc) or disc tracks.

Logic timing circuits coupled to the photodiode can register the variation in the distance the light has traveled (when it strikes back the disc surface) and the distance it has traveled (when it strikes an indentation in the disc surface). This difference is detected as a change in the phase shift in the light beam.

As with all digital coded information, the pattern composed of successive pits and lands - relayed as an electronic string of 1's and 0's by the photodiode - can represent much more complex analog equivalents, such as for the present case, the level of the binding between a target and its capture molecule. This information illustrated as pits present on the surface of the disc according to the invention is the result of the binding between the "capture" and "target" molecules.

To successfully communicate by means of nothing than a series of pits in a disc requires computer processing and some already available high-technology wizardry. At no point does the laser's read mechanism ever touch the disc surface; all data is preferably conveyed by reflections of the laser. In a normal audio CD, the laser beam takes a certain amount of time to return when it is reflected off the lands, but it takes longer to travel if it is swallowed up and reflected by pits. The depth of the pit is engineered to be 1/4 the wavelength of the laser light. If the reflected beam from the pit cancels out the beam from the land, a signal transition is obtained. Signal transitions (signaled by the beginning or end of a pit) represent binary 1's. If there is no signal transition, this indicates a binary 0.

One particular feature of commercial CD-drives is their property to read such pits and deliver data at unpriseve 900 Kb/sec, making this laser reflector technology particularly suitable for the reading not only of the registered pits but also the result of the binding.

To maintain synchronization while reading the data patterns, the CD drive uses self-clocking mechanism that is commonly found in hard disc drives, which is called *Run Length Limited.* Because data exists within finite divisions on the spiral track, each data division extends approximately 300 nanometers, the CD-microcontroller can produce regular clock signals by synchronizing to the speed of the disc rotation and the occurrences of transitions. Although many forms of data storage use a 8-bit sequence for storing data bytes, the normal CD requires a 14-bit pattern to avoid creating combinations of 1's and 0's that would prevent decoding of the stored data. This modified form of storage is called EFM (Eight-to-Fourteen Modulation). An additional 3 bits called *merging bits* act as separator between the 14-bit part, resulting in a 17-bit pattern to represent a single 8-bit byte of data.

Another significant division of data at the bit level is the *frame*, which consists of 588 bits. The frame encompasses a collection of bits : some of them signify data, others allow the laser to be synchronized with the spinning of the disc and still others contribute to the error-correcting capabilities within the CD equipment. Of this collection of bits only twenty-four 17-bit units (408 bits altogether) can be translated into 8-bits bytes. Many additional bits are needed to convey the information contained in a mere two-dozen data bytes.

Another feature of this invention is the possibility to identify the area covered by the capture and target molecules and the various sections of the surface. In one particular embodiment the synchronisation of the biological reading is obtained from the detection of a starting label radius present or deposed or engraved on the disc. The calculation of the distance and/or the area of the targets and the different sections is then performed from this starting radius at each turn of the disc.

In another embodiment, each section is surrounded by a chemical or physical label which is detected by the reader and used for the identification of the area covered by each capture probes and each sample analyzed in each section of the disc area. Each area is then converted into target and sample identification given the appropriate template. In one embodiment the template of the arrays present on the disc is present as numeric information on the same CD. In one particular embodiment the localisation of the area on the computer screen after digitalisation of the biological data gives the identification of the targets trought the information present as numeric information on the disc.

The disc according to the invention can be in any "external" shape form. As above-mentioned, the form of said disc is preferably circular or elliptic, but its external shape form may be for instance hexagonal, octagonal, in the form of a square or a triangle which allows the rotation of said disc along a central axis (A).

The disc according to the invention may correspond to the standards of CD-ROM XA, CD-DVD, audio CD, CD-ROM, CD-I, recordable CD and photo or video CD (CD-ROM and CD-I bridge), etc. Said CD standard may differ according to the type of data storage, accuracy and amount of information.

Preferred embodiments that are most advantageous for manufacturing and operation of the compact-disc of the invention have dimensions within one or more of four pre-existing formats :
- 3-inch compact disc (CD), having a radius of about 3.8 cm and thickness of about 1 mm,
- 5-inch CD, having a radius of about 6 cm and a thickness of 1 mm,
- 8-inch CDV (commercially termed a "Laservision" disc), having a radius of about 10 cm and a thickness of 2 mm, and
- 12-inch CDV disc, having a radius of about 15 cm and a thickness of 2 mm.

The lifespan of data stored on a magnetic tape when covered by ranges from about 6 to 12 years. Estimates for recordable compact-disc lifespans generally suggest a century of stable data storage or even 200 years as requested for the FDA approval CD.

The lifespan of the specific disc according to the invention is shorter and usually limited by the metal corrosion and the possible denaturation of the capture molecule fixed upon the solid surface. The data stored on CD may exist in the familiar concentric circles (referred to as tracks) of the hard disc drive world or in a continuous spiral like the phonograph records of days past.

One particular property of the compact-disc and its encoded information is the tracking system. Different systems exist in commercially available CD-recorders in order to control the movement of the entire optical pick up in it, radially across the disc, and to search for any one of up to 20000 different radial tracks present on the CD. Said technique can be advantageously adapted for the reading of the signal that is the result of the binding between the target and the capture molecules. The reading of the signal and the reading of the pre-registered information can be done by the same device or by two different reading devices, which can be the same laser beam reading device or two laser beam reading devices.

The correction of the radial tracking (identification of a binding upon a capture molecule by a light beam) is performed by using specific systems, as the one described in the publication The CD-ROM Handbook, 2d Ed. (Chris Sherman Editor, Intertext Publication, McGraw-Hill Inc.). The CD-drives also use special device servomechanisms in order to position the laser's reading head.

Preferably, the disc incorporates microfabricated mechanical and/or optical control components on platforms made from, for example, plastic, silica, quartz, metal or ceramic and/or microchannels as described in the document WO97/21090. For the purposes of this invention, the term "microfabricated" refers to processes that allow production of these structures on the sub-millimeter scale. These processes include but are not restricted to photolithography, etching, stamping and other nano or microtechnological means that are familiar to those skilled in the art.

Additional descriptions of a CD solid support are given in the following publications: The CD-ROM Handbook, 2d Ed. (Chris Sherman Editor, Intertext Publication, McGraw-Hill Inc.), The Complete Recordable CD Guide (Lee Purcell & David Martin, Sybex Editions), Digital Audio and Compact-disc Technology, 2d Ed. (Luc Bart, Luc Theunissen and Guido Vergult, Sony Service Center Europe, Ed. BH Newnes).

"Target" and "capture" molecules can be any kind of biological and chemical compounds, which are able to create a binding (or a specific fixation) between each other, said binding or the result of said binding can be detected by a reading device, preferably by using a light beam, preferably a laser beam.

Preferably, said "target" molecule is present in a sample selected from the group consisting of blood, urine, cerebrospinal fluid, plasma, saliva, semen, amniotic fluid, air, water, soil or disrupted biological matter.

Preferably, said "target" and "capture" molecules are synthetic or natural molecules selected from the group consisting of nucleic acids, antibodies, saccharides, lipids, peptides, proteins, lectins, catalysts, receptors, agonists or antagonists of receptors, fluorophores, chromophores, chelates, haptens, ions, molecules having different chiral structures, new synthetic chemical macro-molecules obtained by combinatorial chemistry or other functionalized macrostructures, portions or a combination thereof.

A "capture molecule" according to the invention means a molecule that is not a "cleavable capture molecule" does not comprise and need a cleavable spacer as described in the document WO98/01533, to allow or to permit the detection of the binding between a target molecule (or analyte) and a capture molecule. According to the invention, the simple binding between a target and a capture molecules allows thereafter the formation of a signal that was not previously present (i.e. at the surface of the disc) and that can be detected directly or indirectly by a reading device using a light beam, preferably a laser beam, without requiring any specific cleavage of the capture molecule.

The target or the capture molecules according to the invention are advantageously detected and/or quantified in order to obtain the monitoring, the study and the characteristic behaviors of pathogenic, therapeutical, toxic and/or other improved properties of a target molecule.

The antigens/antibodies binding allows antigens or antibodies detection and are used in diagnostic tests based upon RIA and ELISA detection methods. The ligands/receptors have mainly been developed in pharmacological research for the screening of new molecules (agonists, antagonists or reverse agonists of receptors). Nucleotidic sequences detection has been highly developed through the increased knowledge of the sequence of numerous genes and the development of amplification, hybridization, separation and purification techniques (see, e.g. J. Sambrook, E.F. Fritsch and T. Maniatis, Molecular cloning: laboratory manual, Cold Spring, Harbor Laboratory Press, Cold Spring Harbor, New York).

A first popular detection and amplification method of nucleotide sequences comprises the step of a Polymerase Chain Binding (PCR) (US patents 4,683,195 and 4,683,202) or other amplifications, such as the Ligase Chain Binding (LCR) (Wu and Wallace, 1989, Genomics 4: 560-569), transcription based amplification systems (Kwoh et al. 1989, Proc. Natl. Acad. Sci. USA, 86: 1173-1177) or Cycling Probe Binding (CPR) (US patent 5,011,769). The mRNA is retrotranscribed into a cDNA and either analysed as such or amplified by one of the here above mentioned methods.

Nucleotide sequences detection, quantification and recording by signal of said detection and/or quantification, is obtained after the hybridization of a nucleotide sequence on a capture probe (either by a single or sandwich hybridization) and with the labeling of one of the sequences which give rise to a detection signal, the changes of which can be recorded by the reading device according to the invention.

Many detection methods have been applied to DNA sequences (detected by their own absorbance at 260 nm or by their fluorescence in the presence of ethydium bromide). The use of radioactive labeling like ³²p incorporated into the nucleotidic sequences allows a sensitive detection, but is not recommended for routine assays due to improved safety constraint legislations.

In addition, nucleotide sequences can be labeled by molecules (for example fluoresceine, rhodamine, ruthenium or lanthanide chelate which can be directly detected) or labeled in such a way as to bind enzyme conjugate. A labeling is obtained with the use of biotin or an hapten and an enzyme conjugated to streptavidine or a corresponding antibody. Advantageously, different signals can be obtained according to the product of the binding for detection methods in colorimetry, fluorescence, chemi- or bio-luminescence. For example, peroxidase and alkaline phosphatase give a colored product with the use of TMB (Tetramethylbenzidine) or 5 bromo-4 chloro-3-indolylphosphate as substrate. A light emission can be obtained with the use of luminol or AMPPD (3-(2'-spiroadamantane)-4-methoxy-4(3'-phosphoryloxy) 1,2 dioxethane) as substrates.

The DAB (Diaminobenzidine) can be transformed into an insoluble product after oxidation by a peroxidase catalyzed binding. Pyruvate kinase can also be used for the production of ATP which is transformed by luciferase in order to obtain a detected light (bioluminescence detection method).

Advantageously, new technologies like Mass Spectrometer analysis (MALDI or MALDI-TOF) plasmon surface resonance or optical waveguides may be used for the detection of non-labeled target molecules binding and the follow-up of binding kinetic (Stimpson et al. 1995, Proc. Natl. Acad. Sci. USA: 92, 6379-6383).

The capture molecules are preferably fixed at specific intervals on a CD in order to allow specific discrimination between each binding made between a specific capture molecule and its target molecule by a light beam detection device or another device. According to the invention the capture molecules are located in a specific area of the disc which does not comprise any groove or pre-registered information in order to avoid any false positive which can be the result of a signal upon pre-registered information.

The location of the "capture" molecule on the external surface of the compact-disc can be addressed by conventional physical methods using microlithographic and/or micromachining techniques of incubation which will maintain the non-cleavable capture molecules at certain locations where they will be fixed. An alternative method is obtained by using photoactivable chemical groups which allow the fixation of capture molecules at specific treated locations, such as a portion of said external surface, treated by a light beam (such as a focused laser beam) or treated by selective ion beam or selective plasma treatment (see document WO96/15223).

Advantageously, the surface of the compact-disc according to the invention also contains data which allows the disc to be read in an laser-based CD reader (information usually stored as a series of pits located in the disc grooves and which are necessary to localize the capture molecule on the surface of the disc). This can be obtained through the presence of appropriated pits and lands or protruding indentations equivalent to the pits in the disc grooves.

The addressing (binding) of the non-cleavable capture molecule on the surface is best obtained using such data and using a laser beam which is part of the overall device and if possible the same laser beam source used for reading the CD information.

The binding of capture molecules on the disc surface is obtained by using conventional methods based either on covalent or non-covalent bindings. The preferred embodiment is the covalent binding at one of the molecule extremity, which allows a stable binding link and an homogeneity in the non-cleavable capture molecule presentation at the disc surface for the binding to a corresponding target molecule.

A photoactivable chemical group like azidonitrophenyl which can be be bound to the extremities of any molecule bearing a free amino group by using for instance heterobifunctional reagent like sulfosuccinimidyl 6-(4'-Azido-2'- Nitrophenylamino hexanoate (Pierce, Rockford, IL, USA). Such a photoactivable group will react only at the place of illumination such as a laser beam (Dontha, N. et al. 1997, Anal. Chem. 69: 2619-2625) and in this way the fixation of a specific probe can be well address on the disc. Other chemical fixations exist like the 5' - Phosphate end group fixation of nucleic acid on the amine by carbodiimide or trapping in polypyrrol polymers. Polymeric surfaces can be carboxylated and aminated in order to allow the fixation of most of the biological molecules through bindings well known in organic chemistry (Zammatteo et al. 1996, Anal. Biochem. 236: 85-94).

One particular way to physically address the capture molecule is to take advantage of the centripetal force arising from the rotation of the disc. The liquid is projected through inlet pits to enter the disc and then convoyed through microchannels and valves until binding chambers (WO97/21090).

The binding of the target molecule upon its capture molecule is obtained in standard and reproducible conditions which are now well known either for the nucleotide hybridization (preferably under standard stringent conditions), for the antigen/antibody bindings, for the receptor/ligand binding or other molecules interactions like protein/nucleotides or chemical/chemical molecules recognition.

Preferably, said molecules contain (either by nature or per modification) a functional chemical group (primary amine, sulfhydryl, aldehyde, etc.), a common sequence (nucleic acids), an epitope (antibodies), a hapten or a ligand group, to allow said binding.

The binding between the target molecule and its capture molecule may depend on the specific affinity of the molecules, on the allosteric properties of each molecule, on their ionic environment, on the ionic charge of the molecule and the possible covalent reaction between the target and its capture molecule. Preferably, said conditions are the ones already described in the literature for each type of binding, and can be adapted by the person skilled in the art in order to avoid positive or negative false detections.

Furthermore, the (compact) disc according to the invention having fixed upon its surface the capture molecule may comprise either a protective layer possibly made of organic compounds which allow or improve protection and stabilisation of the capture molecules such as a layer made of proteinic and/or saccharidic compounds like albumin, disaccharides (such as trehalose, etc.) or a layer to improve the binding of capture molecules upon the solid support surface.

The composition of such a layer is adapted by the person skilled in the art according to the specific capture molecule used. If necessary, such composition can be adapted in order to allow the laser beam to read through said layer without difficulty and to detect the binding between a target molecule and its capture molecule or the result of said binding. If necessary, said layer may be omitted before or after the binding between capture and corresponding target molecules.

The above capture molecules can be also located in cavities, chambers or channels present on or in the disc or on the disc surface as long as the surface can be scanned/read by a detector and a signal resulting from the binding obtained and recorded. Said capture molecules can be present also upon a second material which is a strip of plastic upon which the binding between the target and the capture molecules has already been performed and which is thereafter fixed upon the disc side for its specific reading.

Advantageously, each strip can be loaded with several different capture molecules that will react specifically with the same sample or different samples to be analysed. Thereafter, the signal can be read individually or simultaneously upon the same disc. A classical disc like a compact disc could be able to handle 20 or more of such strips.

The addressing (binding) of the capture molecule on the surface is best obtained using data and a laser beam (which can be part of the overall reading CD device) and if possible the same laser beam source is also used for reading the CD information.

The binding of capture molecules on the disc surface is obtained by using conventional methods based either on covalent or non-covalent bindings. The preferred embodiment is the covalent binding at one of the molecule extremity, which allows a stable binding link and an homogeneity in the capture molecule presentation at the disc side for the binding to a corresponding target molecule.

A photoactivable chemical group like azidonitrophenyl which can be be bound to the extremities of any molecule bearing a free amino group by using for instance heterobifunctional reagent described in the document WO99/35499.

The labelling and detection of a signal resulting from the binding between the target molecule upon its corresponding capture molecule are also described in the document WO99/35499 incorporated herein by reference.

The disc according to the invention is used in a diagnostic kit, in a diagnostic and reading device which allows automatically the lecture of a sample preparation of a chemical or biological compound, possibly by a previous treatment of said chemical or biological compound (such as genetic amplification of a nucleotide sequence).

Preferably, said device is a system that combines multiple steps or substeps within an integrated system such as an automatic nucleic acid diagnostic system, which allows the steps of extraction, purification of the nucleotide sequences in a sample, their possible amplification (through known genetic amplification methods), their diagnostic and possibly their quantification.

Advantageously, the disc is part of a processed platform formed of several components being either assembled or disassembled according to the need and/or to the advancement of the overall steps necessary before performing the detection on the disc.

The disc dedicated to the reading of the reaction can be temporarily separated from the other part(s) of the disc and the different parts are manufactured and can be handled separately. Furthermore, in the present invention, the registered data and the biological data are present on two separated supports (90,91). The binding of the target molecules are performed on the part 90 bearing the capture molecules. After reaction and/or labelling the different parts are then assembled together and read on the disc reader device. The method of rotating the disc platform while reading the biological target fixed was found to be particularly useful and sensitive when compared to static measurement.

The target binding may be also performed on plastic, cellulose or nitrocellulose parts which are first handled for target fixation and are then fixed or bound onto the disc for the reading purpose.

In an alternative embodiment, the binding reaction is performed on a support (90) having a disc shape or a symmetrical shape necessary for being read while turning, but containing no registered data. After reaction with the target molecule, this support is inserted onto a disc bearing tracks and registered data. This disc is preferably a mini-CD (a writing or rewriting CD). The mini writing CD is used for recording the necessary information for a reading purpose.

In another preferred application, the biological or chemical data obtained from the binding target molecules are stored as registered numerical information. The same disc platform containing at least some identical information present as both numeric registered data and as biological or chemical data is one specific embodiment of this invention.

Preferably, the target and capture molecules are nucleotide sequences and the method may involve the step of providing target nucleic acid sequences (possibly, copied, amplified or not), and hybridising said nucleic acids to an array of capture nucleotide sequences immobilised on a surface being part of a disc format support, where array comprising more than 5 different nucleotides and each different nucleotide is localised in a determined region of the surface, the density of the different oligonucleotides is greater than 5 different oligonucleotides per cm² and identifying and/or quantifying the hybridised target nucleotide. In a preferred embodiment several samples, (preferably more than 3) are analysed on the same disc.

In another embodiment the arrays contain capture nucleotide probe specific for the identification and/or quantification of the genotype of organism.

The detection on the array may be preceded by a genetic amplification (PCR) for all the possible target molecules requested by the analysis, each of these target nucleotide molecules having at least one or several capture sequences for a detection on the disc. The PCR primers are consensus primers for all or some of the targets to be detected. The number of primers are preferentially limited for having high amplification yield of all target molecules even in a complex biological sample. The preferred number is below 5 but higher are possible if well designed and requested by the necessity of amplification of all the analysed target molecules.

The present invention is also well adapted for detection of multiple homologous nucleotide sequences, coming from different or the same organism, by hybridisation on single stranded capture nucleotide sequences.

In one particular application, the array presented upon one side of the disc allows the detection of the organism species together with the genus and the species.

In another embodiment, the method involves the step of providing a pool of target nucleotide sequences comprising RNA transcripts of one or more target genes, or nucleic acids sequences derived from the RNA transcripts, hybridising said pool of nucleotidic sequences upon an array of capture nucleotide sequences immobilised on the side of the disc opposite to the side comprising registered data, the array comprising more than 5 different nucleotides and each different nucleotide is localised in a determined region of the surface, (the density of the different oligonucleotides being greater than 5 different oligonucleotides per cm², and the nucleotide sequences being complementary to the RNA transcripts or nucleotide sequences derived from said RNA transcripts) and the step of quantifying the hybridised nucleotide sequences in the array of said disc side.

The method may involve that the capture molecules present on the disc platform are antigens and the targets molecules are antibodies to be detected (or vice-versa) wherein microarrays comprise more than 5 different antigens and each different antigen being localised in a determined region of the disc surface, the density of the different antigens is greater than 5 different antigens per 1 cm² and wherein many arrays are present on the disc side opposite to the side comprising registered data.

Another aspect of the invention is a method to translate library of biological compounds present in solution in one or more multiwell plates into an array of bound compounds onto a side of a solid support being a side of the disc of the invention. Said biological compounds are for example but not limited to cDNA libraries or proteins libraries. In a particular application, said solutions containing each of the component of the library is present in wells of multiwell plates (such as in 96, 384, 1536 or more well plate format) and are thereafter spotted onto said binding solid support surface in micro droplets so as to bind on limited area.

Another aspect of the invention is a method to translate library of chemical compounds present in solution in one or more multiwell plates into an array of bound compounds onto a solid support being a side of the disc of the invention. Said chemical compounds are synthesised using parallel chemistry in solution present in multiwells plates and after synthesis, said solutions of the wells are thereafter spotted onto said binding solid support surface in micro droplets so as to bind on limited area surface, thus making an array. The binding capacity of the compounds may be tested in one single experiment by an incubation of the surface of one side of the disc with a receptor and then determining the binding capacity of the chemicals present on the surface. Binding of said molecules to specific (possibly orphan receptors) is an indication of the chemical being a potential drug.

Similarly, molecules acting as agonist or antagonist of a receptor are bound to said solid support surface being a side of the disc of the invention and chemical components are tested for a potential inhibitory effect on this binding. The synthesis of these chemicals incorporates in one of these steps a molecule having a reactive group, which can be used for specific binding upon the said surface. The reactive group can be present as such or be protected during the synthesis and then deprotected before the end of the synthesis. The reactive group can bind by itself to the said surface or be activated for the binding to occur. The preferential groups are amino, carboxylated, sulphide or hydroxyl groups being present onto the chemicals and allowing a binding to the surface support. A preferred method of preparing the disc for a chemical library analysis is to spot the chemicals obtained from multiwell plates (either of 96, 384, 1536 or more wells). Chemicals are also possibly synthesised on microbeads which can be spotted or spread on the disc surface.

Advantageously, each of the chemicals is identified on the disc array by its position and this information is inserted as registered data in the numeric information part of the disc surface (preferably opposite side surface). One particular interest of this invention is to use the array of these chemicals as a storage facility for a chemical library and used it afterwards for testing receptors (determine the binding capacity of these chemicals or for screening new possibly orphan receptors). Supports like the disc side conserved in appropriated solutions with covalent bound molecules can be used for months (and probably for years) after their preparation without any loss of binding capacity.

The method of the invention is adopted for a screening of molecules which bind to the target molecules or allow other compounds to bind to a capture molecule instead of a known target molecule, and the receptor, enzyme, protein, antigen or chemical agonist/ antagonist of a target molecule to a receptor. The binding capacity of receptors in protein-chips as well as the disc side surface of the invention allows a screening of clinical compounds and potential drug with physiological or therapeutic effect.

Such method is also particularly useful for testing chemicals or proteins (including enzymes) which interact with a second binding site of the target molecule (said protein or enzyme are activated in such a way as to allow this binding). Protein-protein interaction like the one observed in kinases cascade in cell activation or transcriptional factors and other regulatory proteins (or enzymes) may be also used, detected and/or quantified as new possible compounds including drugs interacting with such binding or with these proteins activation.

One unexpected finding of this invention is the possibility to use a recordable disc, preferably a compact disc (CD or DVD), for making the assay on a side of a disc like a CD without interference with the reading and recording of the CD. The reading (and recording) are performed by a laser beam oriented on one surface of the CD while the target signal is being obtained on the other side surface. Recordable CD contain layer sensitive to laser light in order to provoked bumps but the recorded laser is reflected on a layer of metal, usually silver or gold so that the reading laser light does not cross the CD. In this way the other side of the CD is available for performing target molecule detection and the obtained signal can be read by an independent reading device without any interference from the numeric reading performed with a laser beam located on the other side of the disc. The two surfaces of the same disc are then considered as independent testing, reading and recording area. CD with the two surfaces being available area for storage of the two detections information and detection are one of the preferred embodiment of the invention. The two area of the CD cover partially or preferably totally the same locations of the disc.

The use of a recordable CD was also found very easy if a very uniform silver coating was put on the disc surface so that light was reflected without or with very little diffusion. The presence of the target together with a silver precipitate under the form of crystals provoked a strong light diffusion and diffraction which could be recorded with detection system. In this particular embodiment of the invention the CD surfaces served as two area, the first one being devoted to read the numeric information and the other one the target signal.

In one particular embodiment the recordable CD are used in order to transform the target data into numeric ones. The readings of the target on the CD is transformed by the appropriate software into numeric data and recorded on the CD, preferentially the same CD.

In another particular embodiment a CD serve as substrate for storage information in the form of capture molecules being present on the CD. Numeric information present as a succession of one-zero signals are transformed into data bytes. In the most simple embodiment a capture molecule is spotted in a line according to a succession similar to the zero-one of the numeric information and is read and transformed in bytes in the same way in order to be transform into supra information such as words, numbers, music, software or data. In another embodiment several different molecules are spotted in different lines, each of the molecules corresponding to specific bytes, the number of lines depending on the complexity of the bytes. Each of the bytes corresponds to a specific capture probe or its target. The capture molecules are present in a bank and are spotted on the CD. In this way the succession of numeric bytes are transformed into a succession of capture probes. The different capture probes are spotted in the same line if hey can be read in a distinctive manner.

Advantageously, the capture molecules are DNA sequences. For example 256 DNA bytes are spotted into 256 lines which are present on the same disc and read together. The radial alignment of the bytes gives the succession of the bytes and represent the supra information required such as the words, numbers, music, software or data. Capture probe alignment is observed by hybridisation with appropriate target molecules. In another embodiment, the presence of the capture is observed by using directly labelled capture molecule. In this embodiment, capture probes are target molecules. In a preferred embodiment, the DNA sequences are present in multiwells plates. In another particular embodiment, the DNA bytes are deposit by piezzo, micro or nano-pipettes, ink jet spotters. Each of the DNA bytes is spotted from a pipette being located on a different line.

In a particular embodiment the sequences are small enough in order to get the necessary information. A 4 bases long DNA sequences existing possibly as 256 combinations of 4 bases while a 5 base long DNA has 1024 combinations and so on. The DNA bytes can thus be limited in number making them particularly attractive for information storage. The DNA based information storage is much more efficient than the numeric data since they are 4 base system compared to a 2 based system for the numeric ones (0/1). Therefore, the content of recorded information upon a small surface is extremely increased and the disc according to the invention can be used as a biological RAM (biological Random Access Memory). Said CD can be used for DNA based storage or numeral information, which may exist upon the registered area of the disc or in a memory of another support (RAM of a personal computer, a diskette, etc.) can be transformed into said biological based information storage and reciprocally (possibly on the same CD or not), the two information types being preferably read by a readable CD or DVD reader.

The same disc platform may contain several separated parts, one of them at least having capture molecules for target molecule detection and these parts can be handled separately and then reassociated for reading the results.

The registered numeric data give to the CD-reader, the necessary information for the CD-drive, to control the speed and/or the location of the laser beam on the CD tracks. The adjacent or consecutive pits present on the CD give information on the localisation of the bound capture molecules and/or of the arrays. In a specific embodiment the registered information present on the CD also gives the information to the arrayer for the location of the deposit of the capture molecules on the disc.

The locations of the disc containing the biological data are preferably not covered with a reflective layer such as given by the gold or aluminium so as to lower the background and maximise the detection and quantification of the presence of the target on the capture probes of the arrays. The absence of metal coating is especially useful for performing fluorescence detection.

The disc material containing the biological data are preferably composed of non-fluorescent material. The polymer and the material added into or on the polymer show not or low fluorescence at the emission of the light emitted by the fluorochrome used for target labelling. One of such fluorescent free polymer is composed of polymethylmethacrylate or polyolefine. However, the disc 5 may contain small areas where non-fluorescent materials as mentioned here under are present or are inserted. One of such materials are polymers developed by NUNC (Roskilde, Denmark) or small pieces of glass.

The fluorescent labelling of the target molecule is preferably performed by an incorporation of a fluorochrome linked to dNTP during DNA or RNA copy or amplification. The preferred fluorochromes are (but not limited to) CY3, CY5 and CY7. Targets are preferably first labelled with a molecule moiety and then recognised by a second fluorescent binding molecule. Such pair of binding molecules used in biological assays are (but not limited to) biotin-streptavidin or antigen-antibodies coupling pairs.

Magnetic labelling of target molecules is made possible by the use of metal deposit. The most easiest labelling is the use of magnetic microbeads as provides by Dynal. The beads are present as streptavidin coated, thus reacting on the biotin targets. Determination for the presence of the magnetic molecule is best made using floppy or hard disc reading devices.

In magnetic determinations, a rigid or floppy disc contains magnetised and non magnetised regions that correspond to bits of data. The magnetisation of these bits is changed by moving the recording head close to the bits. The reading and recording head generated magnetic flux that will either magnetise, demagnetise or leave unchanged the selected regions on the disc. Data can be recorded on selected locations, by controlling both the magnetic flux on a write head and the relative position of the magnetic disc with respect to the write head. By using standard formats for subdividing and labelling the disc, recorder data can be rapidly located and retrieved by a read head.

Hard discs are typically made of rigid material such as aluminium. The read and write head never touches the magnetic hard disc but flies over at submicrons distance. The hydrodynamic bearing surface is created by rapid rotation of the disc and overcomes the force of gravitation, thus preventing the head from crashing over the disc. The disc wear is thus reduced to a minimum and the head can be rapidly moved across the disc surface.

Other discs such as floppy disc are made of mylar and are read by a head touching the magnetic disc making obligatory the lubrication of the surface in order to avoid excessive wear.

The spotting of the capture molecules from a solution to the surface of the disc is performed by any appropriate arrayer which dispenses volumes from multiple solutions in the nanoliters range onto the surface of a substrate. The arrayers may use mechanical spotting on predefined regions of the surface or the solution is delivered through microchannels on defined or predefined regions. One aspect of the invention is to obtain a translation of a rectangular matrix of information into a circular one. The solutions to be used for spotting on the disc are placed in 96, 384, 1,536 or more multiwell plates and arrays of between 10 and 100 or even 1,000 , 10,000 or more spots are formed on the disc. Solutions containing the capture molecules are present in rectangular shape reservoirs such as the multiwell plate format and after spotting on the disc and making the reactions, are read by a combination of turning and radial movements of the reading head. Corrections are preferably made after the data acquisition. Circular correction is already performed using a theta based robot for spotting the solution onto the disc.

The size of the spots are preferentially between 0.010 and 1mm but spots as low as 0.001 mm or even lower are also useful especially for electric based detection.

In another preferred embodiment of the invention, core of the disc is made of organic material preferably composed of polycarbonate, polymethylmethacrylate, polyethylene, polypropylene, cycloolefine, polystyrene or polyacrylate polymers.

The surface of the solid support for the capture probe binding has a chemical composition different from the core of the disc. The surface is covered by a layer, preferably made of organic compound, which allows the binding of the capture molecule and/or improves the protection and stabilisation and/or detection of the cross-reaction between the target molecule and its capture molecule and protect the registered data. The preferred polymers are acrylate based or containing acrylate or polyacrylic varnish. The preferred layer is transparent to the light may resist to temperatures of between 10 to 100°C and binds covalently the capture molecules. Polymers are either a physically deposit on the disc surface or better spin coated in the form of chemicals containing monomers or oligomers still able to polymerise. The polymerisation is obtained by known methods including, but not limited to free radical initiators, UV or other light activation preferably by RadCure method. Preferentially, capture molecules are covalently fixed on the surface containing chemical groups able to bind them within a minute period and without any third coupling agents. These reactions include, but are not limited to aldehyde/amine, acrylate/amine, isothiocyanate/amine or thiol/thiol binding. Coupling can be obtained between chemical groups like the amine/carboxilic, thiol/amino, alcohol/amino and other couples of functional groups (see Zammatteo et al 2000 (Anal. Biochem. 280, 143-150 and the Pierce catalogue on bifunctional reagents for biological molecules coupling and attachment).

The acrylate/polyacrylate polymer is a preferred layer since it responds to the characteristic here above mentioned for the formation of a layer on the disc, the binding of the capture molecules, the treatment of the disc and the reading of the results.

The olefine containing polymers may also be first oxidised in order to form aldehyde groups on the surface of the disc thus allowing the covalent binding of the capture molecules. Preferably, the oxidation step of the surface of the solid support allowing the formation of aldehyde functions is obtained in the presence of low concentrations of permanganate and periodate in a buffered aqueous solution. Oxidation in aqueous solutions prevent damages to the polycarbonalte polymer. Aminated capture molecules are covalently fixed on the aldehyde groups through Schiff base formation which is then reduced with NaBH4 for stabilisation of the linkage. Aldehyde groups are also preferentially obtained by plasma deposition of acetaldehyde or acrolein vapour onto the surface of polymers.

The arrays are present at specific intervals on the disc platform in order to allow the identification of the incubated samples on each array. The disc also contains a signature in order to identify the position of the arrays compared to other portions of the surface. The simplest signature is a lateral lign or bare code on the disc surface which is detected by the reading device. The position of each array is then reconstituted after the reading and attributed to one or more given sample(s). The data corresponding to each array are then analysed separately using an image recognition software for the recognition and quantification of the spots of the array and possible substracted from background noise for instance by the identification of homogeneous parts of an image after having been merged into two classes used as training sets.

Data resulting from the presence of the target compounds are extracted by a reading device for microarray possibly present on a disc platform or a compact disc reader based on the combination of two movements: a first one being a lateral movement and another one being a circular movement, the combination of the two resulting in an efficient scanning of the array and the data associated with the presence or not of the target molecule in the discrete area or specific location of the disc surface containing specific capture molecules. The dynamic of the rotating movement while reading the data increases the sensitivity of the target detection of the microarray compared to a static observation such as obtained with a CCD camera.

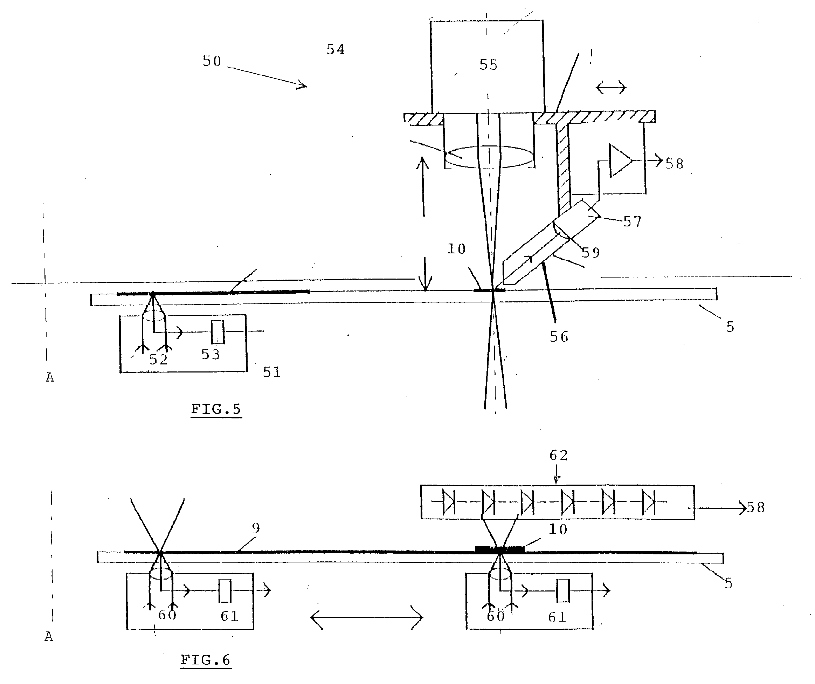

Preferably, the optical detection system which can read said binding may comprise a photo-diode which can detect a small light beam and which moves according to a one dimension axe so as to cover the radius of the disc. Combined with the rotation of the disc, such focused photo-detection scans the entire surface of the disc and so to assay for the target molecule present at any location on the disc. A preferred detection device is a photo-diode of the commercially available CD readers which are used for music, video or software CD (see Fig. 5).

The photo system can be servocontrolled in order to stay in focus to the detection surface. If a second optical detection system is provided for the detection of the signal, it can also be servocontrolled or linked to the other one for its control, or it may receive from the first one data in order to adjust its focus and its tracks to the disc. The data received from the consecutive reading of the disc surface can also be stored in a computer, reformated if necessary and analyzed for the definition of spots localization.

The photometric signal is obtained once the binding between the target molecule and its capture molecule allows the formation of a photometric signal. Advantageously, the detection and/or the quantification of said binding (binding of the target molecule to its non-cleavable capture molecule) is based upon the principle of CD binary detection system using the variation in the laser beam reflection. A perturbation in the laser reflection is obtained when the laser beam detects in the groove a pit.

According to a first embodiment of the present invention, said pit is advantageously a precipitate which is a result of the binding between the target and the non-cleavable capture molecules.

According to another preferred embodiment, a perturbation in the laser reflection can also be obtained by a corrosive attack upon one or several layers of the compact-disc. For instance, the binding between the target molecule and its capture molecule may provoke a limited modification of the layer which will form an indentation in said layer. Such indentations in the layer are usually called "mounds" or "bumps", but are also identified as negative pits (see document Recordable CD Guide, Lee Purcell & David Martin, Sybex Editions). These perturbations will be detected by the laser beam as pits which differ from lands. The binding between the target molecule and its capture molecule can also be detected and quantified directly by a light emission obtained only when said binding takes place.

According to another preferred embodiment of the present invention, the binding of the target molecule upon its capture molecule allows the binding of one or other marker molecules which produce a light or radioactive emission through a chemo, bio, fluoro and/or electroluminescence light system or will create a magnetic and/or electric field which could be detected by specific reading devices (see Figs. 1 to 5).

These systems or methods can be based upon the use of specific enzymes (peroxidase, alkaline phosphatase, pyruvate kinase, etc.) which allow or improve light emission and binding detection.

One may use a labeled target molecule or a second labeled reactive marker bound to the target molecule have reacted with the capture molecule. This labeled molecule (marker) can be a first member of a binding pair (such as nucleic acid molecule in a sandwich hybridization assay binding a complementary sequence, one or two of them being labeled to biotin or on hapten and similarly an antibody/antigen sandwich binding may use similar labeled reactive compounds. After a washing step, the first member (biotin or hapten) can react with an enzyme-conjugate streptavidine or antibodies which are then considered as the second member of the binding pair. Enzymes such as peroxidase, alkaline phosphatase, pyruvate kinase or other dehydrogenases can be used.

One selects specific substrate for said enzymes in order to obtain an insoluble product. For example DAB can be oxidized in the presence of peroxidase and form an insoluble product. This product will precipitate upon the non-cleavable capture molecule, and such precipitate will form limps or mounds on the surface of the disc according to the disc surface that will be illuminated by the (laser) beam. The reflected (laser) beam intensity will be lowered when illuminating the precipitate and a perturbation in the (laser) reflection can be obtained. Such a perturbation is analyzed by the photosensitive detection device as a pit upon the surface of the disc.

If detected by light transmission through a transparent part of the disc, the presence of the precipitate will show an absorbance that can be measured.

Another insoluble product is obtained when colloidal metal like gold is used, for example bound to streptavidin. The colloidal gold catalyzes the reduction of silver (Ag) to form Ag-precipitate where the binding is obtained (WO 00/72018). Silver deposit can either diminish the (laser) light beam reflection when superpose to gold or aluminum layers usually present on a disc but on the other side can reflect or diffract the light if no other metal is present. This precipitate being opaque to the light can also be detected by absorption of the light in a transmission assay through the disc. Metal precipitates are also conductors of electricity or have magnetic properties which can be detected as such since they strongly differ from the disc support which is usually an inert polymer, like polycarbonate.

It is also possible to use microbeads for labeling which bear binding molecules (second binding pair) which allow the recognition of a first binding pair attached to the target molecule. These microbeads will be located on the surface of the disc where the binding of the target molecule and its non-cleavable capture molecule has been obtained. These beads will diffract or absorb the (laser) light beam and will create a perturbation in the (laser) reflection. These perturbations in the (laser) reflection will be detected by the photosensitive detection device and analyzed as the pits upon the surface of the disc.

According to another embodiment of the present invention, the detection is obtained by a labeling of the target molecule (with a fluorescent marker). The (laser) light beam will scan the compact-disc surface and analyze the fluorescence recorded. Many fluorescent markers associated with the target molecule are available, like fluoresceine, phycoerythrine, rhodamine or lanthanide chelates, which can be easily labeled upon nucleic acids, antibodies or microbeads for a direct or indirect labeling of the target molecule.

The recorded signal can be read either as a binary signal or as an absolute value. The binary signal is advantageously quickly processed as an electronic computerized data and analyzed by appropriate software. This software will convert this information into data which can analyze the detection obtained and quantify the binding between the target molecule and its non-cleavable capture molecule.

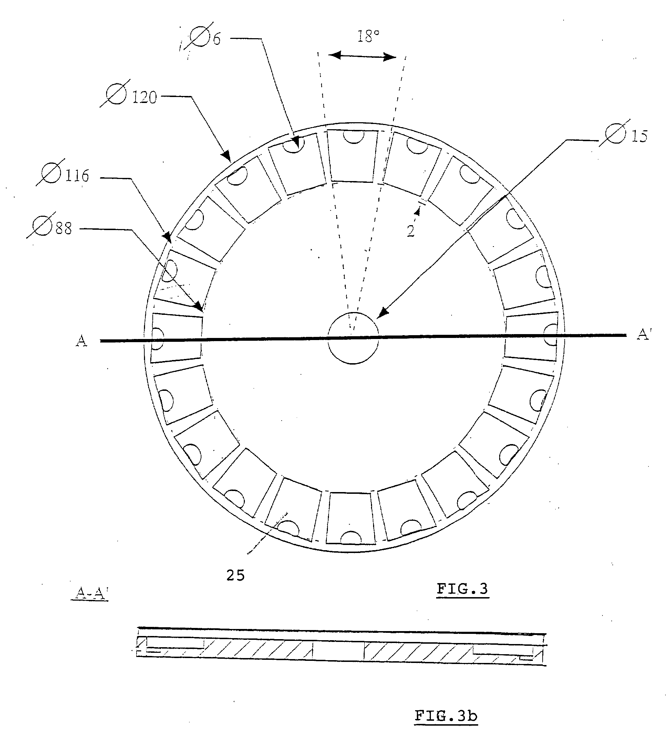

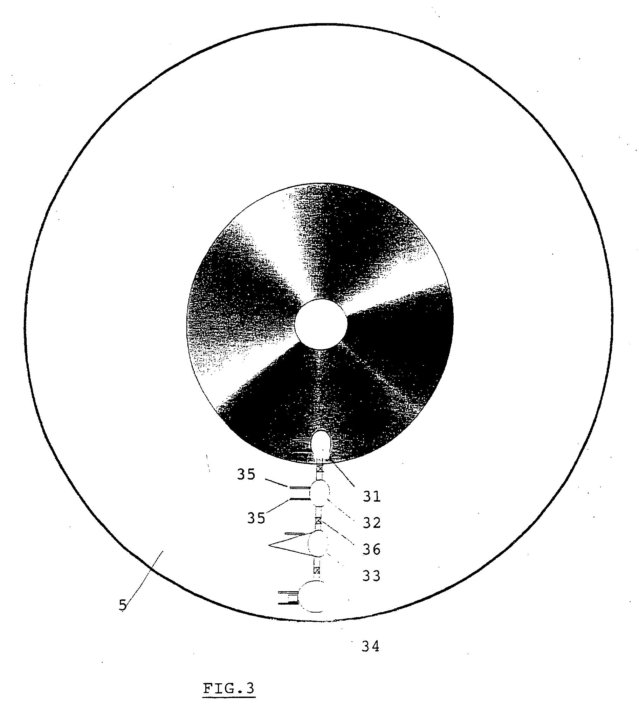

Preferably, the disc according to the invention may comprise additional pits, preferably in the groove adjacent to/or opposite to the face surface (side) comprising the non-cleavable capture molecule, which give information about the type, the quantity and the specificity of said adjacent non-cleavable capture molecule (see Figs. 3 to 5).

According to a specific embodiment of the present invention, the disc according to the invention bears a bound oligonucleotide capture nucleotide sequence so as to allow a detection, amplification and possibly quantification of a target nucleic acid sequence upon a same solid support (the surface of the disc according to the invention). In an alternative form of execution, the disc comprises PCR primer in solution in a chamber, or bound to the disc surface in order to obtain the production of amplicons and binding of amplicons upon one surface of the disc, which allows thereafter their detection (according to the method described by Rasmussen et al. 1991, Anal. Biochem. 198: 138-205).

In a further preferred embodiment of the invention, the disc platform is rectangular in shape having preferably the size of a credit card. The registered data and tracks 2D are located on the inner part of the mini-disc-card, while the detection of the target molecules is performed on the outside of the tracks (even in the corners of the support), possibly separated by an aluminium layer.

The surface bearing the capture molecules is one of the two faces of the disc comprising one or the two faces covered with a polymeric layer. In another embodiment, the disc is shaped in a way as to provide other surfaces for detection of the target molecules. The disc may contain cavities located inside the disc so that incubation of the target molecule solutions can take place.

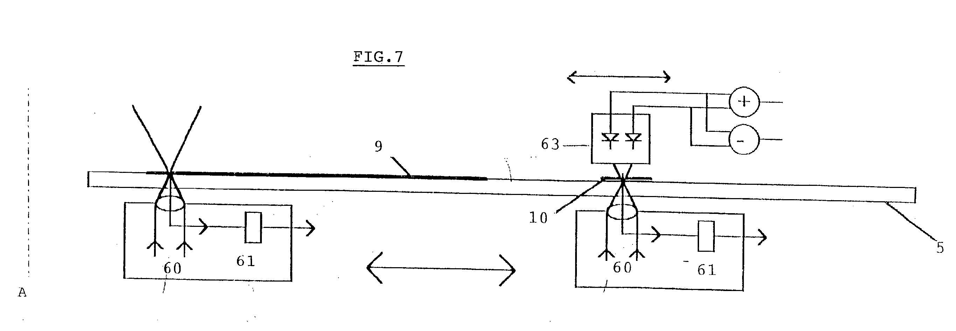

Preferably, the arrays are present upon a support 5, but separated by small enclosures provided by hot embossing method. Hybridisation chambers 22, preferably closed chambers, may be fixed on the disc surface in order to obtain small incubation chambers for the target solutions to be in contact with the disc surface (Fig. 7) and forming advantageously a DNA micro-array 23 which can be read and analysed by classical DNA-array reader.

The same disc platform may contain several separated parts, one of them at least having capture molecules for target molecule detection and these parts can be handled separetely and then reassociated for reading the results.

The disc platform is preferably composed of different parts which allow to perform some or all of the steps necessary before the detection of the target molecules binding upon corresponding capture molecules. A molded material may be placed on the surface of the disc providing the necessary chambers for incubation of the targets with the capture probes. A particular design of such molded platform for making 20 closable chambers of incubation 25 is presented in Fig. 2. Advantageously, the molded chamber is removed after the incubation and the disc processes for washing, labeling and reading the results.

Several chambers (31, 32, 33, 34) may be located in connection with each arrays in order to perform the hybridization (34) the retrotranscription, the amplification step (33) and/or the dilution of the solution (32) and/or the DNA or RNA extraction (31) (Fig.3). These chambers are connected with each other and with the outside media or recipient with tubings (or microchannels) and valves (35, 36) in order to introduce or to eliminate the necessary reagents and solutions. Valves 36 are open by using the centrifugal force of the turning disc 5. In another embodiment the valve 36 is open by melting closing material present in said valve or channel either by the application of a local heat material or air or by a steady illumination with a laser beam upon said material.

The molded chambers are located on a heating plate equivalent or equivalent heating device. The chambers can be heated at a constant temperature like necessary for DNA or RNA hybridization on the capture sequences. The preferred temperature of hybridization is between 40 and 70° (but temperatures lower than 40° and higher than 70° may be also used, depending of the characteristic of the molecules). The temperatures of hybridization may be optimized by the person skilled in the art as a compromise between the rate of reaction, the sensitivity and the specificity of the detection.

The heating device is preferably a Peltier type system for performing fast changes in temperatures necessary for the DNA amplification necessary for PCR. The preferred device controls the three temperatures necessary for the denaturation, annealing, elongation typical of the amplification. Each of the temperatures and the length of incubation is optimized according the well-known procedures of the PCR technology. Other temperature cycles are also possible under control of the Peltier type device.

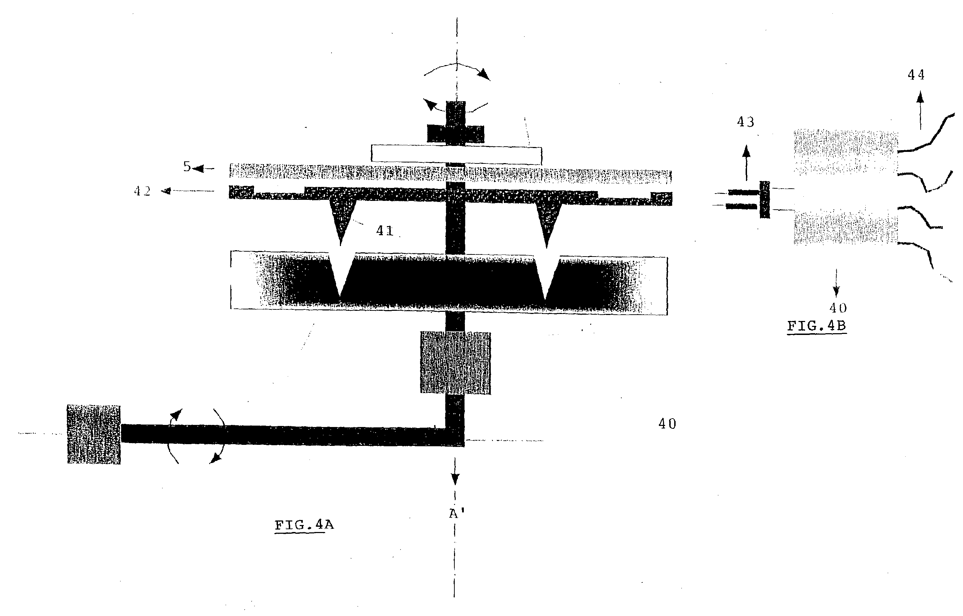

Preferably, the disc platform is inserted in an automate 40 for handling the various steps of the process (see Fig. 4). The automate can contain all or only some of the features described here above or equivalent devices for performing the same process. In the automate there is an axe perpendicular A' to the discs surface along which devices can rotate of 180°, so as to locate the incubation chambers 41 formed by the molded material 42 either on the upper or inner part of the disc. The chambers are located on the upper part of the disc for the incubation with the targets and washing steps. The disc is then rotated so as to be present on the upper part of the chambers, before a colorimetric labeling (such as using the silver deposit (WO 00/72018)). The inventors have discovered that this process lowers advantageously the background of the detection. The automate also contains all the necessary needles (43) valves, tubing (44) and pumping devices for the washing and incubation necessary for the binding of the target molecule on its corresponding capture molecules and their labeling. Once obtained, the disc with both information are then read in the reading device.

The disc 5 is read, by an optical disc reading system 50 composed of a double illuminating and double detecting device. A preferred example of such machine 50 is presented in the figure 5 and in example 6. A first illuminating 52 and detection 53 device is the normal CD or DVD reader 51, which allows the reading of numeric information engraved on the CD. The registered data are read as 0-1 data through variations of the reflection of the light from pits present on or in the CD. The reading of the registered data gives to the CD-reader 51, the necessary information for correcting the focus of the reading head on surface of the pits and to follow the tracks. The reader also identifies the CD.

The registered numeric data give to the CD-reader, the necessary information for the CD-drive, to control the speed and/or the location of the laser beam on the CD tracks. The adjacent or consecutive pits present on the CD give information on the localisation of the bound capture molecules and/or of the arrays. In a specific embodiment the registered information present on the CD also gives the information to the arrayer for the location of the deposit of the capture molecules on the disc.

Reading of registered data is based on the reflection of a laser based beam. When encounters the flat surface of the disc, the laser beam is reflected back by the presence of a reflective layer on the surface usually made of gold or aluminium. Registered disc may contain a series of small pits cut into the disc surface. When the laser passes onto the pits, or when entering or leaving the pits the reflection of the light may decrease. Recording of the light change is recorded by a sensor, converting these changes into one/zero information, which sequence is then used for any type of information storage. The scan 54 for the presence of the target molecules is performed with the second illuminating 55 and reading 56 device with preferentially the light focussed on the surface where the target molecules are present. The illumination and detection for the target molecules can be located on the same size of the disc as described in the figure 6. In this case reflexion or diffraction of the light is measured. The two devices can also be present on opposite sites of the disc in order to obtain a light transmission assay. Modification of the detected light is a measurement of the presence of target molecules on their capture molecules. The detection is preferably performed by a photodiode 57 comprising a lens (59) and the analogic signal 58 recorded, while the disc is turning. The two devices are independent from each other and read two different kinds of information.

The optical disc reading system is composed of a double detector and one illuminating light. A description of a second light based reading system is given in the figure 6. The light is emitted by the laser beam 60 of the CD-reader 61 which first scans the numeric information of the disc 5 present as registered data. The same laser beam 60 also scans the parts of the disc bearing the biological or chemical data and binding of target chemical or biological molecules. The presence of the target molecules is then obtained by the use of the second detector 62 recording the variations of the light. The second detector is preferably located on the opposite site of the light emission and the variations of the light in the target locations are obtained by an absorbance of the light (by a series of detectors 62 arranged in line with the laser illumination (Figure 6)). Location of the signal is obtained by asservissement of the light position or by the identification of the photodectors recording the transmitted light.

A second detector 63 may follow the radial movement of the laser beam on the opposite side of the disc and record the variations of the transmitted light (Fig. 7). The various area of the CD which contain the registered information and the biological data are scanned sequentially while the disc 5 is turning.

The presence of the target molecule is characterised by a change in the light absorption at the given area of the disc measured by the second detector 63. This second detector 63 is preferably physically or electronically bound to the light emission device and the second detector is able to move lateraly in order to scan the surface of the disc 5, while turning.

Engraving the target molecule presence data onto numeric information on the same disc is possible using a conventional recording "write or record" CD system. Recording of numeric data on CD is performed using specifically layered CD and a laser based engraving machine. A high power laser enters the medium at the substrate and passed trough transparent substrate until reaching an expension layer. The expension layer absorbed the light and raise its temperature, thus resulting in a increase pressure. The increase of the expension layer temperature, then diffuses into the retention layer which softened and thus allowing the expension layer to flow into the area and creating a well defined bump. The retention layer follow the contour of the bump and protrude in a soft reflective layer. Reading of the bumps is performed essentially as described here above for recorded information engraved into the CD at the time of its manufacture. The write and record CD system may be adapted to read the presence of the target molecule as exemplified here above.

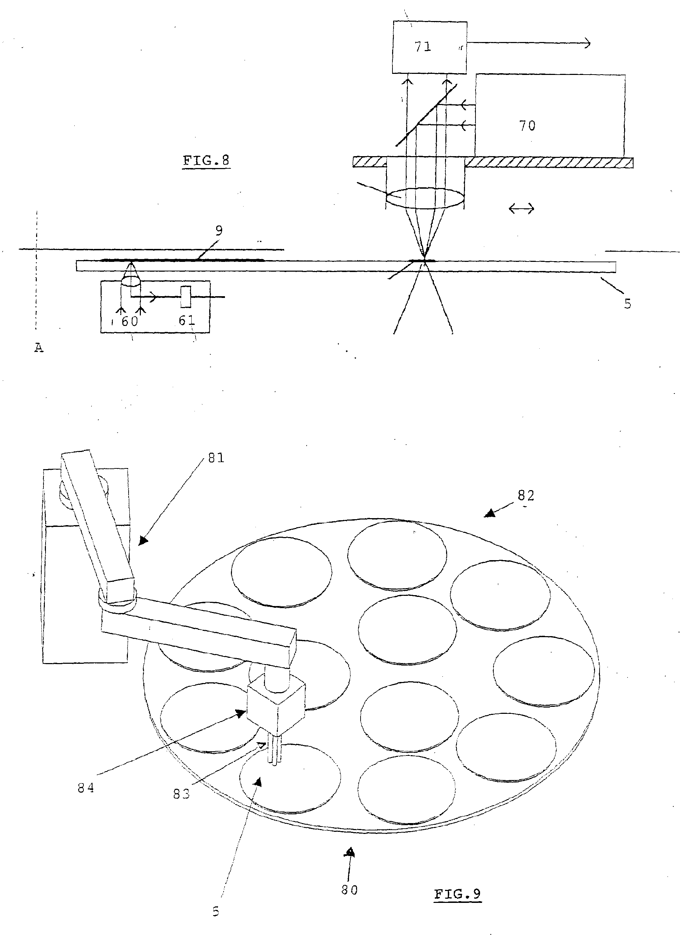

The preferred fluorescent reading device is provided in figure 8. It is composed of the excitation of the fluorescence by a laser beam 70 having a wavelenght corresponding to about the maximum exitation of the fluorochrome. The emitted fluorescent light is detected in a sensitive light detector (7). (The emitted light intensity is detected and measured trought a photomultiplier.) A filter may be introduced into the emitted light path for selecting the appropriate light wavelength increasing the sensitivity of the target detection and/or measurement. The excitation and reading devices are physically or electronically coupled. The two devices move in a laterally controlled movement in order to scan the entire disc while turning and registering the biological data. The fluorescent light emission may be timely interrupted or pulsed in order to allow reading of the emitted fluorescent with lower or no interference with the excitation light.

Another preferred machine 80 is presented in figure 9. It is composed of a theta controlled robot 81 and a rotating table 82, both movements being coordonated.

The spotting allowed by circular arranged spots (but square arrangements are also possible). The solutions are present in mutiwell plates and taken with a pin, or a series of pins 83, for spotting on the disc 5. The arrayer located its pins upon the first or the designed wells and then by touching the solution removed a small quantity of liquid attach on the extremity of the pin. The arm 84 of the robot 8 then move in both a rotating and translateral movement such as to position the pin 83 above the disc 5 surface. The desired precision is obtained by a position feedback mechanisms using a closed loop system. The electromechanical mechanism will preferably have a repeatability of less than 5 micrometers. The pin 83 will then move down and reduce its speed when approching from 0.05 mm or less from the substrate. The height above the substrate is preferably determined by moving the dispenser taward the substrate by small increments until the pin touches the substrate. The number of increments are recorded and correspond to the specific distance. This measurement is repeated over the plate and corrections for unhomogeneity of the plate is then obtained. The solution is preferably taken from the wells in a ring and deposited on the disc with the pin

The solution is preferably incorporated into a capillary and spotted as microdroplets with a piezzo controlled device. The localisation of the head is obtained by controlled 85 system as above mentioned. An appropriate distance of between 5 and 50 micrometers and preferable 10 micrometers are used between the head and the surface of the substrate. Then a drop of between 0.1 and 5 nanoliter is dispenced onto the surface.

### Examples

### Example 1: Detection of protein Chips on CD with colorimeric labeling: direct detection of spotted antigen

### Protein chips construction

Streptavidin was diluted to a final concentration of 100µg/ml in a spotting buffer Borate 0.05M pH 8, glycérol 40 %, NP40 0.02 % and spotted as an antigen at the surface of a polyacrylate based polymer coated CD. The CD contained layers of a r-CD (Recordable CD) which can be read and recorded by a laser beam directed on one side of the CD (the down part in a classical CD-reader) and the spottting was performed on the other side of the CD. The spotting was obtained with solid pins of 0.250 mm diameter and the spots were around 0.35 mm diameter final After 3 washes with phosphate pH 7.4 0.01M + 0.1% Tween 20, nonspecific binding sites were blocked with PBS containing milk powder at 0.1% for 1h at 20°c. The CD characteristics, mainly, the number of arrays, of spots and their identification were written on a part of the recordable : CD layers. Some parts were still free for further writing of the final detection results. For detection of antibodies, the CDs chambers were incubated for 1h at 20°c with rabbit anti-streptavidin ranging from 1.2pmole to 12amoles in 100µl in PBS + milk powder at 0.1%. After 4 washes of one minute with a 10 mM maleate buffer containing 15 mM NaCl and 0.1% Tween pH 7.5 ( washing buffer) CDs were incubated for 45 min at 20°c with a conjugate of anti-rabbit IgG/gold particles of 10nm diameter (diluted 100 times) in100 mM maleate buffer containing 150mM NaCl.

CDs were washed 5 times in the same washing buffer as before and then incubated for 10 min in the Silver Blue detection solution (AAT Namur) for obtaining the silver cristal precipitation. The CDs were finaly washed in water before being read in the CR-Reader.

### ELISA in multiwells

Multiwell plates coated with streptavidin (Roche) were used as a support. The wells were incubated for 1h at 20°c with 100µl of rabbit anti-streptavidin diluted from 1.2pmole to 12amole in 100µl in PBS with milk powder at 1/%.The wells were then washed 4 times with a 10 mM maleate buffer containing 15 mM NaCl and 0.1% Tween pH 7.5 ( washing buffer).The plate were incubated for 45 min at 20°c with conjugate anti-rabbit IgG/peroxidase labeled diluted at 1/100 with 100 mM maleate buffer containing 150mM NaCl. Wells were washed 5 times in the same washing buffer as before and then incubated for 10 min with TMB ( in the dark). The reaction was stopped with 100µl of stop solution and the samples were readed at 405nm in a ELISA reader.

For the protein chips, a detection limit of the of anti-streptavidin of 120amol was obtained when a solution of 100µg/ml of steptavidin was used for spotting on the CD.

In the ELISA, a limit of 120amol of anti-streptavidin was obtained.

For both methods the same limit of measurment of antibodies was obtained, but with a detection surface of around 35 mm² for the multiwell while a surface of 0.096 mm² and a mutliparametric detection for the protein spots on the chips.

### Example 2: Detection of protein Chips on CD with colorimeric labeling: detection of antigen, IgE, in solution

The sandwich detection was performed as follows : RAT IgE antibodies (from mouse) were spotted on CD in a spotting buffer Borate 0.05M pH 8, glycerol 15 %, NP40 0.02 % to a final concentration O.1mg/ml. After 4 washes of 2 minutes with phosphate pH 7.4 0.01M + 0.1% Tween 20, nonspecific binding sites were blocked with maleate buffer 100 mM pH 7.5 containing 150 mM NaCl milk powder at 0.1% (blocking buffer) for 1 hour at 20°c. The CDs chambers were incubated for 1h at 20°c with RAT IgE (diluted 10 000 times in blocking buffer). After 4 washes of one minute with a 10 mM maleate buffer containing 15 mM NaCl and 0.1% Tween pH 7.5 (washing buffer) CDs were incubated for 1 hour with RAT IgE antibodies (from GOAT) (diluted 1000 times). After 4 washes of one minute with a 10 mM maleate buffer containing 15 mM NaCl and 0.1% Tween pH 7.5 (washing buffer) CDs were incubated for 45 min at 20°c with a anti-GOAT-IgG conjugate to gold nanoparticles of 20nm diameter (diluted 100 times) in blocking buffer.

CDs were washed 4 times (for 2 minutes) in the same washing buffer as before and then incubated for 10 min in the Silver Blue detection solution (AAT Namur) for obtaining the silver cristal precipitation

Applications to other antigens. The method is applied to the following tumors antigens : the Carcinoembryonic antigen (CEA), the alpha foeto-protein (AFP) and the Cancer Antigens (CA 15-3, CA 19-9, Ca 125, CA 125 11, CA 195, CA 72.4, CA 549),the ACE, the hCG, the NSE,, the Tg, ferritin,b2 microglobulin, erythropoietin, the squanous cell Carcinoma (SCC), the Prostate Specific Antigen (PSA, PSA II, III US), the Tumor Associated Glycoprotein 72 (TAG 72) and the Tissue Polypeptide Antigen (TPA).

IGE can be detected either as antigens or as antibodies by using specific allergens on the CD.

Assay on xenobiotics or drugs on which the antibodies are available are also usefull in microarray on CD, the cannabis, amphetamines, cocaine and benzodiazepine, among others.

### Example 3: Detection of auto-immune antibodies on CD

Antigens were spotted on the CD as described in example 2. The proteins were spotted with a pin based arrayers in order to obtained spots of around 0.4 mm. The antibodies possibly present in the sample were incubated for 1h and process for detection with the silver blue detection after having reacted with a conjugate of antihuman IgG/gold particles. The results are obtained as darkening of the spots which are positive. A program conbining image analysis software and quantification give the values for the presence or not of the different antibodies corresponding to the spotted antigens.

Applications on the detection of autoimmune desease by the identification of the antibodies is very well adapted to the protein chips on CD since a large number of possible antibodies can be screened simultaneously for their possible presence in the patients fluids ; a non limitative list of such antibodies is presented in table 1. These included the detection of the anti-neutrophil-cytoplasmic antibodies (ANCA) such as the Proteinase 3(PR3) for the diagnostic of the Wegener's granulomatosis, the Myeloproxidase (MPO)for the diagnosic of the Churg-Strauss syndrome, polyarteritis nodosa, microscopic polyangiitis and Rapid Progressive Glomerulonephritis. Other autoantibodies usefull to detect are the anti-cell nulcei (ANA) (mRNP/Sm, SM,SS-A,SS-B,Scl-70), the anti-mitochondria (AMA), the anti-liver antigens, the anti-Parietal Cells( PCA), the anti-Neuronal Antigens (Hu,Yo,Ri), the anti-endomysium .

Other applications are the detection of different antibodies as anti-thyroglobulines, anti-thyroperoxidases, the anti-insuline, anti-erythrocytes, anti-gliadine, ani-HLA A,B,C and DR, anti-thrombocytairs, anti-tissue, anti-spermatozoides, anti-nuclear, anti-cytoplasmic antibodies. In diabetes, usefull assays are the detection autoantibodies such as IA-2 autoantibodies, the anti-Islet Cell antibodies (ICA), the anti-insulin antibodies (IAA)and the anti-GAD antibodies.

Table 1 presents additional examples of capture molecules that may be bound to the surface of the disc.

### Example 4: Magnetic detection of DNA or protein on CD

Detection of hybridized DNA or protein on CD support can be achieved by magnetic process. Biotin bound to DNA or antibodies can be recognized by streptavidin conjugated to ferro-fluid (Immunicon, Hungtinton Valley, PA, USA). This conjugate is magnetic or paramagnetic according to the size of ferrite nucleus of the ferro-fluid and can then be detected in a magnetic field.

### Example 5: Detection of several bacterial species and their genus by DNA microarrays present on the CDs

### Bio-CD™ spotting

Aminated DNA capture probes were spotted on a compact-Disc (Bio-CD™) on locations covered with an acrylate based polymer( UCB-Chemicals, Bruxelles, Belgium). These locations were located on the outside of the disc and not covered by the registered information. The spotting solution was Borate buffer 0.1M pH 8.

Concentration of capture probe may vary from 150nM to 1µM. All capture probes are 5' aminated. The sequences of the probes are proposed in the patent PCT/BE/01/00053. After spotting,the Bio-CD™ was washed 2 min in a 0.2% SDS solution, then twice in water and finally 5 min in boiling water.

### DNA purification from culture samples and culture conditions

*The Staphylococcus* strains used in this study *(S. epidermidis, S. haemolyticus, S. hominis and S. saprophyticus) were ibatined from the ATCC* and from clinical samples (Prof.J-L Gala, UCL, Bruxelles) Bacterial strains are grown medium in aerobic conditions overnight at 37°C from single colonies in LB. A aliquot of overnight culture is pelleted by centrifugation (5000 x g, 5 min). This pellet is resuspended in 300 µl of lysis buffer (50 mM Tris HCl pH 8.0, 100 mM EDTA, 150 mM NaCl, 1 % SDS) containing 100 mg lysostaphin (Sigma, St. Louis, Mo) and 100 µg of RNase and incubated at 37°C for 30min. Another incubation, in 200 µg of proteinase K (Boerhinger, Mannheim, Germany) at 37°C for 30 min and boiling for 5 min, is necessary to achieve the lysis reaction. Lysate was centrifugated at 4000 g for 5 min and DNA is extracted from 200 ml of supernatant by adsorption on Nucleospin C+T columns (Macherey-Nagel, Düren, Germany), according to manufacturer's instructions. DNA is eluted in 200 µl of sterile water and stored at -20°C.

### DNA purification from clinical samples

Clinical specimens (BAL, stumps, AL or ETA) were homogenized in 5 ml of TE buffer (20 mM Tris HCl, pH 8.0, 10 mM EDTA) containing 2% (w/v) SDS. The homogenate (1.5 ml) was then centrifugated for 5 min at 7500 xg. The cellular pellet was washed once with TE buffer, lysed in the presence of 1% (v/v) Triton X-100 and 50 µg of lysostaphin (Sigma Chemical Co., St. Louis, Mo), and incubated for 15 min at 37°C. Lysis was completed by adding 100 µg of proteinase K (Boerhinger, Mannheim, Germany). The lysate was incubated for another 15 min at 55°C and 5 min at 95°C. It was centrifugated at 4000 xg for 5 min. In order to purify bacterial DNA, 200 µl of the supernatant were then filtered on a Nucleospin C+T column (Macherey-Nagel, Düren, Germany) washed and then eluted with 200 µl sterile H₂O, according to the manufacturer's protocol. DNA suspensions were stored at -20°C.

### Duplex PCR for Staphylococcus

Co-amplification by duplex PCR of fem-A and Mec-A genetic markers on Staphylococcus sample (was done using 2 consensus primers for fem A : Apcons31 : TAAYAAARTCACCAACATAYTC, Apcons32 : TYMGNTCATTTATGGAAGATAC, and 2 primers for mec A sequence : Apmec01:TCTGGAACTTGTTGAGCAGAG and Apmec02 : GGCTATCGTTGTCAC AATCGTT at a final concentration of 0.1µM each. The PCR was made in a Tris-HCl buffer 0.075M pH 9, KCl 50mM, MgCl2 2mM, (NH4)₂SO₄ 20mM buffer containing dATP, dCTP, dGTP, at a final concentration of 50µM each and dTTP and dUTP-biotinylated (Roche, Indianapolis, USA) at a final concentration of 25µM each. The PCR ran 5 min at 94°C, then 40 cycles made of 30 sec at 94°C, 45sec at 49°C and 30 sec at 72°C, and finally 10 min at 72°C.

### Hybridization of PCR product

5µl of duplex PCR product were added to 30µl of NaOH 0.1N containing DNA from salmon sperm 100µg/ml, 200pM positive DNA control for positive hybridization and 30µl of 0.7M phosphate buffer pH 7.5 containing SDS 4%. These 65µl of hybridization mix are hybridized onto the array in sealed hybridization chambers for 2 h at 53°C.

### Colorimetric silver detection

After hybridization, the chambers are removed and then the Bio-CD™ is washed 4 times 1min with a maleate buffer 10mM containg NaCl 15mM and tween 0.1% pH 7;5. The BioCD™ is incubated for 45 min at room temperature into a blocking buffer (maleate buffer 100mM NaCl 150mM pH7.5 containing 0.4% caseine) containing a streptavidin-colloïdal gold conjugate diluted 100 times (BBI, England). The BioCD™ is then washed 5 times in the same buffer (maleate buffer 10mM NaCl 15mM pH 7.5 Tween 0.1%). Then Bio-CD is incubated at room temperature for 15 min in the Silver Blue Solution (AAT, Namur, Belgium), rinsed in water, dried 5' at 37°C and read with the Bio-CD reader. Results are digitalized and quantified with softwares included in the workstation.

### Polystyrene beads detection

The protocole was similar to the one for colorimetric silver detection here above, except that the conjugate is made of streptavidin coated polystyrene beads (Dynabeads) diluted 50 times in blocking buffer. After 45 min incubation with the conjugate, the BioCD is washed 5 times with the maleate buffer.

### Double CD player

The reading device is composed of two illuminating and reading systems (fig. 5). A commercial available CD reader was used for reading the numeric information inserted into the CD the speed of the reader was controlled. It is used for reading the numerical information written on the CD and for rotating the CD during the acquisition of the analog biological datas. During this time a constant angular speed of about 1000 rpm is used. A Read and Write CD-reader was also adapted in the same way. A second laser-based reader is intended to read the biological part supported by the CD. It is fixed on the upper part of the numerical CD reader. It consists of a laser beam generator ( 670 nm , 0.8 mW) which illuminates a point perpendiculary to the surface of the CD. The beam is focused by a lens on the surface of the disc in a diameter of 0.05 mm. The focal of the lens is 4cm. The light diffracted by the samples is detected by a photodiode. To avoid parasitic light, the photodiode is inserted in a dark pipe which sees the illuminated point of the CD at an angle of 45°. The outcoming analog signal is amplified between 100 and 1000 times and digitized by a acquisition card (National Instrument) at the rate of 200ksample/sec.

This detector (laser and photodiode) is driven by an electric lateral movement which moves along a radius while the disc is turning, on a lateral distance of 20 mm within one minute. The combination of both the rotating and lateral movements allows to scan the disc and obtain the required datas. A white radius is drawn on the CD .It is seen as the begining of each turn. If the CD support n arrays, the data of each turn are divided into n equal parts and saved into n files. The head moves of 0.05 mm at each turn and the scan of the turn is redone. Each of the files corresponds to an array. The geometry of the array is then corrected since the scale changes with the diameter of each turn. The correction take into account the different lenght of each turn. Coordonates of each point can then be calculated and the image corrected accordingly.

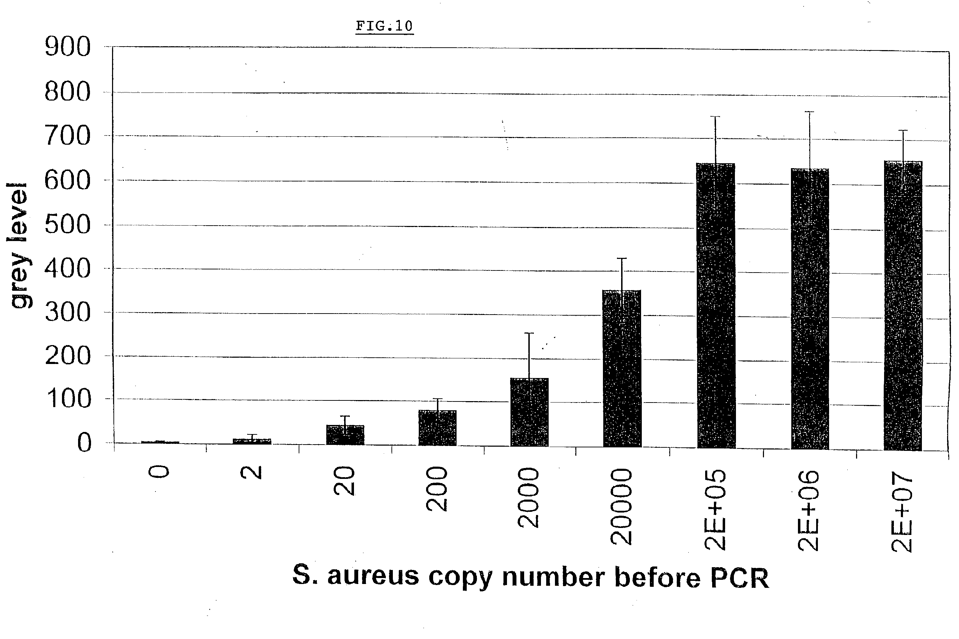

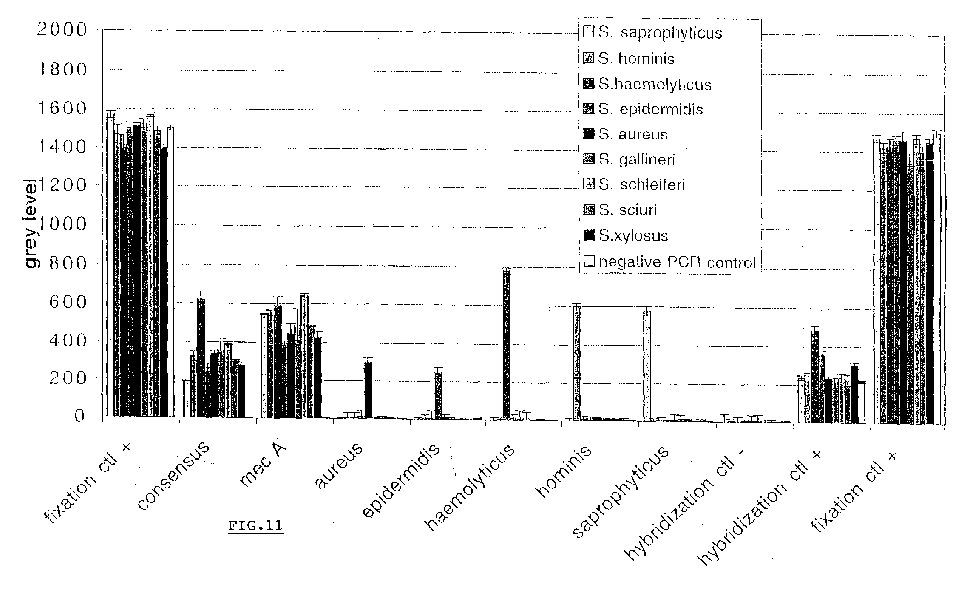

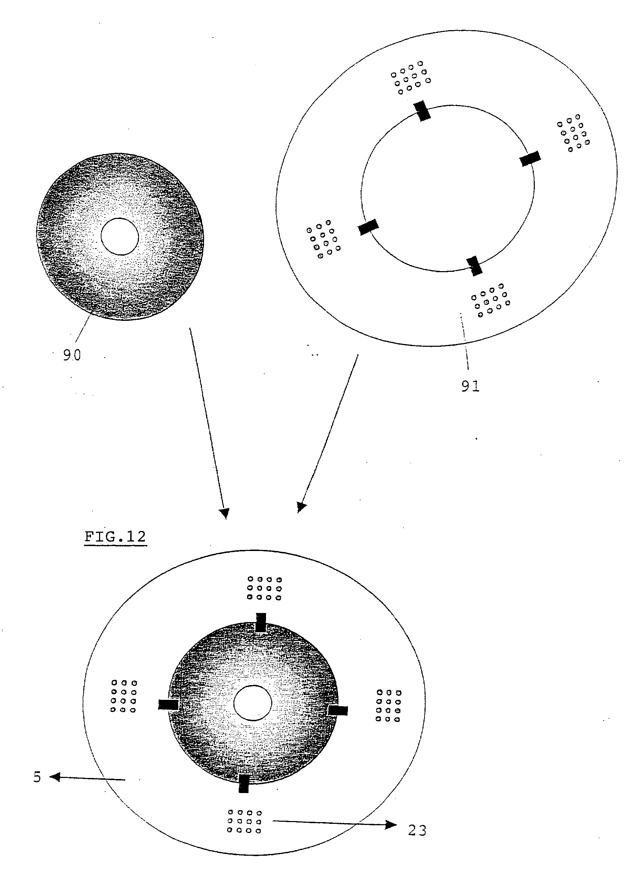

An image recognition program is applied for recognition of the discrete locations bearing the capture probes. Image analysis is then processed by evaluation of the average grey level of the pixels of the spots minus the grey level of pixels surrounding each spot. Data are presented as an excell sheet. The means of quadruplicates are then calculated. The values which overpass a threshold are taken as positive and are processed as values associated with the presence and/or quantification of targets possibly present in the samples. (Fig. 10, 11, 12)

### Example 6: Detection of gene expression on microarrays present on the CDs: exemple of HepatoChips

### HepatoChips Design: Fifty-nine genes microarray

Genes on the Rat HepatoChips™ (AAT, Namur, Belgium) are presented in the table 2. The selected genes are either involved in drug metabolism or may have a potential to act as markers of toxicity. The arrays also include positive and negative controls for the hybridization process, an internal standard control and 8 housekeeping genes (table 3).

### Synthesis of labelled cDNA

Labelled cDNA was prepared using 2µg poly(A)+ RNA isolated from rat liver using the FastTrack 2.0 mRNA isolation Kit (Invitrogen). A synthetic poly (A)+tailed mRNA was spiked to the purified mRNA as internal standard to assist in quantification and estimation of experimental variation introduced during labelling and reading (DeRisi J *et al* 96). mRNA was added to 2µl of oligodT₍₁₂₋₁₈₎ primer (0.5µg/ul) (Gibco BRL), Rnase free water was used to bring the volume to 9µl, and the mixture was denatured at 70°C for 10 min and then chilled on ice for 5 min. The reverse transcription was performed by adding the following components to the annealed probe /template on ice: 4 µl of First Strand Buffer (250 mM Tris-HCl pH 8.3, 375 mM KCl, 15 mM MgCl₂) (Gibco BRL), 2ul of DTT 0.1M (Gibco BRL), 40 units of Rnasin ribonuclease inhibitor (Promega), 500 µM dATP (Roche), 500 µM dTTP (Roche), 500 µM dGTP (Roche), 80 µM dCTP (Roche), 80 µM biotin-11-dCTP (NEN). The reaction mixture was mixed gently by flicking the tube and incubated for 5 min at room temperature. 300 units of Superscript II RT (Rnase H-) (Gibco BRL) was added to the reaction mixture and the reverse transcription was allowed to proceed for 90 min at 42°C. Then an additional 300 units of Superscript II RT was added and incubation was continued at 42°C for another 90 min. The reaction was ended by heat inactivation at 70°C for 15 min. To remove RNA complementary to the cDNA, a treatment with RNase H was performed at 37°C for 20 min following by a heat denaturation at 95°C for 3 minutes and cooled on ice before use (Rajeevan S *et al*, 1999. *J Histochem Cytochem* 1999; **47:** 337-42). No further RT product purification was necessary.

### Generation of the internal standard

The internal standard clone was constructed by the insertion of PCR amplified fragment of the HIV-1 pol region in the vector pSP64 polyA+ (Promega), linearizing isolated plasmid DNA with *Eco*RI and synthesizing polyA+ tailed RNA complementary to the insert from the SP6 promotor (Promega) (Ernest I et *al,* 2001 *J Virol Methods* 2001; **93:** 1-14).

### Hybridization using biotinylated cDNA

The Rat HepatoChip was constructed on the polyacrylate of the CD by spotting aminated single stranded capture probes for the different genes reported in the tables 2 and 3. Two spots per gene have been spotted onto the array, except for some of the control probes. (table 2). The hybridization was performed in a hybridization chamber (Biozym, Landgraaf, The Netherlands) containing the hybridization buffer 'Hepatobuffer' (SSC 2X pH7 and SDS 4%), the total biotinylated cDNA (from 2ug mRNA) and a positive hybridization control (a biotinylated amplicons, provided in the kit at a concentration of 25 nM). Hybridization was carried out overnight at 60°C in a custom slide chamber. Chamber humidity was maintained by small reservoir of 3X SSC. The arrays were then washed four times for 2 min with buffer (10mM Maleic buffer pH 7.5, 15mM NaCl, 0.1% tween) at room temperature.

### Hybrid detection

The presence of biotinylated hybrids on the microarray was detected using the colorimetric based labeling as in example 5. The Bio-CD was incubated at room temperature for 15 min in the Silver Blue Solution (AAT, Namur, Belgium), rinsed in water, dried 5' at 37°C and read with the Bio-CD reader. Results digitalized are quantified with softwares included in the CD-Reader as explained in example 5.

The intensity of each DNA spot (average intensity of each pixel present within the spot) was calculated using local mean background subtraction. A signal was deemed significant if the average intensity after background subtraction was at least 2.5 fold higher than their local background. The two intensity values of the duplicate DNA spots was averaged and used to calculate the intensity ratio between the reference and the test. Very bright element intensities (saturated signals, highly expressed genes) were deemed unsuitable for accurate quantification because they underestimated the intensity ratios and were excluded from further analysis.

The data obtained from different hybridizations were normalized in two ways. First the values are corrected using a factor calculated from the intensity ratios of the internal standard reference and the test sample. A second step of normalization was performed based on the expression levels of housekeeping genes. This process involves calculating the average intensity for a set of housekeeping genes, the expression of which is not expected to vary significantly. The variance of the normalized set of housekeeping genes is used to generate an estimate of expected variance, leading to a predicted confidence interval for testing the significance of the ratios obtained. Ratios outside the 95% confidence interval were determined to be significantly changed by the treatment.

### Example 7: Multiple sample analysis in the different molded chambers present on the same disc platform.

The experimental protocol was identical as described in example 5 with the disc platform covered by a plastic sheet including 20 cavities for making chambers once applied on the CD. 9 duplex PCR products were made on 9 different Staphyloccocus species methicillin resistant and 1 negative PCR on water. The 9 species are : S.aureus, S. epidermidis, S. gallneri, S. hominis, S. saprophyticus, S. schleiferi, S. sciuri, S. simulans and S. xylosus. These hybridizations were made simultaneously on 10 microarrays described on figure 7 on the surface of one BioCD. The amplified targets were incubated in the chambers as described in example 4. The plastic cover was then removed and the disc was processed for the washing and labeling steps. The data acquisition was obtained througt the reader device and the data process for analysis. The results are presented in figure 3.

### Example 8: Steps performed by the automate in the hybridization chamber.

Introduce 100µl or 200µl of an hybridization mix containing detergent (SDS 4%), hybridization buffer and DNA to hybridize.

Incubate this solution for 2H to 12H at temperature between 40°C and 70°C.

Take this solution out of the chamber.
Introduce a washing solution (4ml in 2 min).
Empty the chamber.

Introduce 200µl of conjugate solution (containing non specific proteins and streptavidin-gold) Incubate 45 min at room temperature. Empty the chamber.
Introduce washing solution (4ml in 2 min). Empty the chamber.

Introduce 50µl of Silver blue solution A and simultaneously 50µl of Silver Blue solution B .
Incubate 10 min at room temperature. Wash with 200µl of water. Empty the chamber.

### Example 9: Olefinic oxidation

The olefinic functions present either on polymers were oxidised in the following way. The discs were dipped into a solution of 0.1M Phosphate buffer at pH 7.5 containing 37 mM NaIO₄ and 1.3 mM KMnO₄ under mild agitation during 1h, washed twice with water, dried under Nitrogen flow and stocked under vacuum.

### Example 10: Steps performed by the automate in the extraction, dilution, amplification and hybridization chamber.

The following steps of manipulation presented in example 5 are performed in the successive chambers present on the CD.

### Chamber 1 : DNA extraction

The clinical samples are first homogenized and bacterial lysed by lysostathin treatment. Typical methodology is the following for bacterial DNA extraction from clinical samples. Clinical specimens are homogenized in 5 ml of TE buffer (20 mM Tris HCl, pH 8.0, 10 mM EDTA) containing 2% (w/v) SDS (step 1). The homogenate (1.5 ml) is then centrifugated for 5 min at 7500 xg. (step 2) The cellular pellets are washed once with TE buffer, lysed in the presence of 1% (v/v) Triton X-100 and 50 µg of lysostaphin (Sigma Chemical Co., St. Louis, Mo) (step 3) They are incubated for 15 min at 37°C. Lysis is completed by adding 100 µg of proteinase K (Boerhinger, Mannheim, Germany) (step4). The lysate is incubated for another 15 min at 55°C and 5 min at 95°C( step 5). It is centrifugated at 4000 xg for 5 min(step 6). For further purification, bacterial DNA, the supernatant are then filtered throught a chamber containing silica for binding DNA. The extract is introduced in the upper part of the chamber, go through the silica where the DNA binds(step 7). The other molecules are whashed away( step 8). Then, water is introduced in the chamber to release DNA in solution( step 8). The DNA solution is then pushed into chamber 2 through a pipe after opening of the microvalve (step 9). In this example steps 7 to 9 are performed on the disc. It is also possible to adapt steps 1 to 6 while changing the centrifugation steps into a filtration which can take place in a chamber present on the disc.

### Chamber 2 : DNA dilution

Water is introduced in the second chamber to dilute the DNA sample. A precise volume of liquid (10µl) goes to chamber 3 through a pipe after opening of the microvalve.

### Chamber 3 : PCR amplification

100µl of PCR reaction mix containing primers, polymerase, dNTP and reaction buffer are introduced in chamber 3. The chamber is heated to a temperature of 100°C, then precisely to the PCR required temperatures, in this case 60°C then 72°C and 94°C for 40 cylces. The wall of this chamber is thin (0.3 mm) in order to allow good heat exchange. The whole volume or a part of it goes to chamber 4 via a pipe after opening of a microvalve.

### Chamber 4 : Hybridization on microarray.

The chamber 4 contains the microarray. Once the amplified DNA sample enter this chamber, a hybridization mix (150µl) is introduced to start hybridization. The chamber is heated at 53°C for 2 hours. During the hybridization a micropump introduces small pressure changes in the liquid for mixing.

After hybridization, the whole volume goes out of the chamber and washing solutions and detection solutions are introduced (cfr. example 5).

### Example 11: Target detection trough the reflective layer of a CD with one laser illumination beam and two detectors

The CD has its numerical data in the inner part of the disc. The external part is produced free of dye. It is then coated with a thin reflective layer , so part of the light pass trough the disc, the other part of the light is reflected. The gold layer is protected with a varnish . The varnish supports the biological samples. Some part of the CD can contains numerical information.

The reader device is composed of one illuminating and two detection devices. It is based on a commercially available CD writer (Fig. 6-7).

During the simulation of a writing process, the head scans the surface of the disc, moving slowly from the inner side to the outer side of the CD while the disc is rotating at a well known linear speed.

A fixed light detector (photodiode) is added at the upper side of the CDwriter . It can detect the light coming from the writing head trought all the CD. The intensity of the light is modulated by the sample supported on the upper surface of the CD. This signal is digitized by an acquisition card and stored turn by turn on a hard disc. A black line drawn on the CD shows the begining of each turn. The radial position of the head is given by the number of turns of the CD.The image of the biological samples is then gradualy reconstituted.

The size of the file depends on the resolution of the image. Storage of all turns data with a special resolution of 1.6 um (track pitch of a CD) leads to an image file for the whole Cd of about 6.7 Gigabytes.

Storage of all turns data with a special resolution of 50 um leads to an image file for the whole CD of about 7.2 Mbytes.

Depending of the resolution and the speed off the acquisition card, the time for scanning a whole CD is between a few minutes and one hour.

### Example 12: Description of a fluorescent reading device

The device is composed of two illuminating and detection systems (figure 8). The first one is a laser based reflective detection of the numeric information incoded as pits in the CD tracks. The second one is an analogic head moving lateraly along a disc radius. It contains a laser diode module which gives a laser beam of 650nm wavelenght on the surface of the disc throught a filtering semi-transparent miror and a lens. The fluorescence emitted by the excited CY5 molecules is collected back throught the same lens, passes through the miror and send to a photomultipier (Hamamatsu) where it is amplified. The emitted light is filtered so that the 650nm light is absorbed while the emitted 670 light is measured. These parameters are optimized for CY5 fluorochrome. The signal from the photomultiplier is digitalized by an acquisition card and stored in the computer disc. A software reconstitutes the fluorescent image and the data processed for image recognition of the spots and the data processing for quantification of the signal corresponding to the spots of the array.

**Table 1:**

| List of antibodies useful to be detected according to the invention | |
|---|---|
| Anti Thyroglobulin | ANAcombi |
| Anti Thyroid Peroxidase | Anti-Tissue Transglutaminase |
| ENAscreen | Anto-Prothrombin IgG/IgM |
| Anti-Histone | Anti-Prothrombin IgA |
| Anti-SS-A | Anti-Prothrombin screen |
| Anti-SS-B | ENA-4 Profile |
| Anti-Sm | ANA-6 Profile |
| Anti-RNP/Sm | Anti-GBM |
| Anti-Scl-70 | Anti-dsDNA |
| Anti-Jo-1 | Anti-dsDNA IgA |
| ENAcombi | Anti-dsDNA IgM |
| Anti-Cardiolipin IgG+IgM | Anti-dsDNA Screen |
| Anti-Cardiolipin IgA | Anti-ssDNA |
| Anti-Cardiolipin screen (IgG/IgA/IgM) | Anti-RNP 70 |
| AMA-M2 | Anti-Centromere B |
| Anti-Rib-P | Anti-SS-A 52 |
| Anti-PR3 (ANCA-C) | Anti-SS-A- 60 |
| Anti-MPO (ANCA-P) | |
| Anti-Insulin | |
| Anti-β-2-Glycoprotein I | |
| Rheumatoid Factors, total | |
| Rheumatoid factor IgA | |
| Rheumatoid factor IgG | |
| Rheumatoif factor IgM | |
| Anti BPI | |
| Anti Elastase | |
| Anti-Cathepsin G | |
| Anti-Lysozyme | |
| Anti-Lactoferrin | |
| Anti-Nucleosome | |
| Anti-Phospholipid screen | |
| ANCAcombi | |
| Anti-Gliadin IgA | |
| Anti-Gliadin IgG | |
| Anti-Phosphatidyl-Serine IgG/IgM | |
| Anti-Phosphatidyl-Inositol IgG/IgM | |
| Anti-Phosphatidyl-Acid IgG/IgM | |
| ANAscreen | |

**Table 2:**

| **Sequences presented upon the HepatoChips with their known function and Genbank accession number** | | |
|---|---|---|
| **Gene** | **Fonction** | **Genbank Accesion no.** |
| Bax, Bcl-2 | Apoptosis | U49729, L14680 |
| c-jun, c-myc, Elk-1 | Oncogene | X17163, Y00396, X87257 |
| Cox-2, IL6 | Inflammation | L20085, M26744 |
| Cyp 1A1, Cyp 1B1, Cyp 2B, Cyp | Cytochrome P450 | X00469, U09540, M34452, |
| 3A, Cyp 4A1 | | M10161, X07259 |
| Enoyl CoA hydratase, PPAR α | PP | K03249, M88592 |
| ACO | PP Acyl CoA Oxidase | J02752 |
| Ferritine | Iron Stock | U58829 |
| Fibronectin | Extracellular Matrix | X15096 |
| GADD153, GADD45 | DNA Damage | U30186, L32591 |
| MGMT | DNA Repair | M76704 |
| Glutathione S-transferase | Oxidative stress | K01931, X67654 |
| Subunit Ya ,subunit theta 5 | | |
| GSH Reductase, Heme Oxygenase | Oxidative stress | U73174, J05405, L16764, |
| 2, HSP70, MnSOD, | | Y00497, M16975, M27315 |
| ApoJ, Cytochrome | | |
| C oxidase subunit 1 | | |
| Hepatocyte GF | Growth Factor | D90102 |
| Histone D-acetylase (Hdac1) | DNA Transcription | NM008228 |
| HMG CoA synthetase | Cholesterol Metabolism | X52625 |
| JNK-1, Telomerase, Cyclin D1 | Cell Cycle activation | L27129, U89282, D14014 |
| NFκB, p38, erk-1, c/EBP, Iκβα | Transcription factor | L26267, U73142, M61177, X12752, U66479 |
| Ornithine carboxylase (odc) | Arginine synthesis | J04791 |
| P53 | Tumour Suppressor | X13058 |
| PCNA | Proliferation Cellular Nuclear Antigen | Y00047 |
| Rmdr-1b, Transferrin, Albumin | Transporters | M81855, D38380, V01222 |
| SMP30 | Senescence Marker | X69021 |
| TNF | Tumour Necrosis Factor | X66539 |
| Transforming growth factor-b type II | TGF-beta receptor | L09653 |
| UDPGT1A, UDPGT1A6 | Glucuronyl Transferase | J05132, D83796 |
| Liver +ve control | α2-macroglobulin | J02635 |

**Table 3:**

| **HouseKeeping genes included on the Hepato CD** | | | |
|---|---|---|---|
| **HouseKeeping Gene** | **Function** | **Abundance level** | **Accession number** |
| α-Tubulin | Cytoskeletal protein | High | V01227 |
| Ribosomal protein S29 | Protein synthesis | Medium | X59051 |
| Myosin heavy chain 1 (myr) | Muscle contraction | Low | X68199 |
| Hypoxanthine guanine phosphoribosyl transferase | Nucleotide synthesis | Medium | M86443 |
| Glyceraldehyde-3-Phopshate dehydrogenase(G3PDH) | Glycolysis | High | D16554 |
| Polyubiquitin | Cellular metabolism, development | High | D00036 |
| Phospholipase A2 | Lipid metabolism | Low | X02231 |
| β-actin | Cytoskeletal protein | High | V01217 |

## Claims

1. A method for the detection, identification and/or the quantification of several target molecules possibly present in several samples, preferably biological samples, comprising the steps of :
- allowing a binding between said target molecules of one sample on capture molecules bound upon one section of the surface of a solid support being a disc comprising registered data, said binding resulting in signals,
- said disc surface is divided into at least 3 sections separated by chemical or physical boundaries allowing a detection and/or quantification of said signals, said signals are not obtained through cleavage of said capture molecule;
wherein the signals for the detection, identification and/or quantification of the target molecules are present and/ or read on area present on one side of the disc opposite to the side of the disc comprising registered data, both area covering partially and preferably totally the same location of the disc.

2. The method according to claim 1, wherein the capture molecules of one section are bound on determined locations upon the disc surface according to an array having a density of at least 5 different bound capture molecules/cm² disc surface.

3. The method according to claim 1, wherein the disc surface is covered by a physical layer providing incubation chamber(s).

4. The method according to claim 1, wherein the sections present on the disc are separated by chemical or physical boundaries which are detectable by the reading device

5. The method according to claim 1, wherein the disc surface possesses a radius starting label.

6. The method according to claim 1, wherein the registered data of the disc are binary data, preferably grooved binary data.

7. The method according to claim 1, wherein the disc is a compact-disc.

8. The method according to claim 1, wherein the disc is a recordable compact-disc.

9. The method according to claim 1, wherein the reading registered data allows an identification of the capture probe and/or the treatment and/or the interpretation of the signal resulting from the binding between the capture and the target molecules.

10. The method according to claim 1, wherein the reading of the registered data provides information to the arrayer regarding the identification of the deposit of capture molecules upon a particular area of the disc surface.

11. The method according to claim 1, wherein the disc has at least one portion of the disc surface containing a layer for writing numeric information.

12. The method according to claim 1 further comprising the step of writing results data of identification and/or quantification of the bound target molecules as numeric data, preferably readable by a CD or a DVD reader.

13. The method according to claim 12 wherein the said written results data are numeric data.

14. The method according to claim 1, wherein capture molecules are made of specific molecules corresponding to a limited number of data bytes.

15. The method according to the claim 14, wherein the capture molecules serve as target molecules.

16. The method according to claim 1, wherein registered data are information, preferably bytes, converted into a nucleotide sequences being deposited and/or present on the disc surface.

17. The method according to the claim 14, wherein the alignment of capture molecules is converted into registered information, such as words, numbers, music, software or data.

18. The method according to claim 14 which comprises data bytes present on the same disc as both numeric information and capture molecules.

19. The method according to claim 1, wherein the signal generated by the binding between the target and the capture molecules is either a precipitate, upon the surface of the disc and/or a corrosion of one or more layer(s) of the surface of the disc.

20. The method according to claim 19, wherein the precipitate is either an opaque precipitate or a magnetic precipitate.

21. The method according to claim 19, wherein the precipitate is a deposit of a colloidal metal reagent (metallic precipitate).

22. The method according to claim 21, wherein the metallic precipitate is a silver precipitate.

23. The method according to claim 1, wherein the detection and/or the quantification of the signal is obtained by a fluorescent light emission after excitation of the bound target and capture molecules by a light beam.

24. The method according to claim 1, wherein the detection and/or the quantification of the signal is obtained by an emission selected from the group of a light beam, a radioactive radiation emission or a magnetic field.

25. The method according to claim 24, wherein the emission of a light beam is generated by a marker bound to the target and/or the capture molecule, said marker being selected from the group consisting of chemo, bio, fluoro, electroluminescent and/or radioactive markers.

26. The method according to claim 1, wherein the capture molecules and the target molecules are nucleotide sequences.

27. The method according to claim 1, wherein the capture molecules and target molecules are respectively either antigens and antibodies or antibodies and antigens.

28. The method according to claim 1, wherein the capture and target molecules are respectively either receptors and ligands of said receptors or ligands and their receptors.

29. The method according to claim 1, wherein the capture molecule is a single (or double) strained nucleotide sequence and wherein the target molecule is a ligand of said sequence, such as a transcriptional factor.

30. The method according to claim 1, wherein the target molecules comprise two binding sites, one binding site to its corresponding capture molecule and another binding site for another molecule.

31. The method according to claim 31, wherein said target molecule is an enzyme.

32. The method according to claim 1, wherein the capture molecules are obtained from a chemical or biological library.

33. The method according to claim 1, wherein the detection of the signal allows a characterisation of a chemical compound bound to its corresponding capture molecule at a determined location, as a potential drug acting upon a receptor being its capture molecule.

34. The method according to claim 1, wherein the detection of the signal allows a characterisation of a chemical compound bound at a determined location to a receptor as a potential drug.

35. The method according to claim 1, wherein the detection of the signal allows a characterisation of a chemical compound which acts as agonist or antagonist of the fixation of a ligand to its receptor.

36. The method according to claim 1, wherein the signal observed in a determined location is correlated with the identification and/or a quantification of said target compounds.

37. The method according to claim 1, wherein the detection and/or the quantification of the signal is obtained by reflection, absorption or diffraction of a light beam, preferably a laser beam, or variation of an electromagnetic field.

38. The method according to claim 1, wherein the detection and/or the quantification of the signal is made on a turning disc.

39. The method according to claim 1, wherein the binding between the target and the capture molecules allows the binding of one or more additional molecule(s) upon the target and/or the capture molecules.

40. The method according to the claim 40, wherein said additional molecule is either a microbead or a magnetic particle.

41. The method according to claim 1 wherein the synchronisation of the disc is obtained trought the detection of the radius starting label present on the disc.

42. the method according to claim 1 wherein the localisations of the section area and/or the capture probes area are correlated with the identification of the samples and/or the targets to be detected.

43. A disc comprising registered data, and bound upon its surface, one or more capture molecule which allows a binding with one or more corresponding target molecule to be detected, identified and/or quantified, said binding resulting in a signal; wherein the said capture molecules are bound upon a side of the disc surface being different from the side comprising the said registered data, and wherein the said capture molecules are not cleaved for obtaining said signal.

44. The disc according to claim 43, wherein the capture molecules and/or corresponding target molecules are selected from the group consisting of nucleotide sequences, antigens, antibodies, receptors, ligands of receptors, receptor and enzyme peptides, lipids, saccharides, haptens, fluorophores, chromophores, catalysts, macromolecules obtained by combinatorial chemistry or a combination thereof.

45. The disc according to claim 43, wherein the registered data of the disc are binary data.

46. The disc according to claim 45, which is a compact disc..

47. The disc according to claim 43, which is a recordable compactdisc.

48. The disc according to claim 43, wherein the registered data contain the information for providing a location of the sample and/or the location of the capture molecules on the disc surface.

49. The disc according to claim 43, wherein the capture molecules on the disc surface are a library of molecules, preferably a chemical or biological library.

50. The disc according to claim 43, wherein the capture molecules are disposed on the surface of the disc as arrays of at least 5 spots per cm²/surface.

51. The disc according to claim 43, which bears at least 3 arrays for the analysis of at least 3 samples.

52. The disc according to claim 43, which further comprises upon its surface a polymer layer with aldehyde groups for a covalent binding of the capture molecules.

53. The disc according to claim 43, which further comprises upon its surface a polymer layer with acrylate groups for a covalent binding of the capture molecules.

54. The disc according to claim 43, which further comprises a polymer layer, oxidised prior to the binding of the capture molecules.

55. The disc according to claim 43, which further comprises at least at some specific locations upon its surface one or more non fluorescent layers.

56. The disc according to claim 43, comprising a central portion being a mini-CD.

57. The disc according to claim 43, which further comprises one portion of the disc surface containing a layer for writing numeric information.

58. The disc according to claim 57, wherein said portion is a writing mini-CD.

59. The disc according to claim 58, wherein the said mini-CD comprises data regarding location, detection, identification and/or quantification of a bound target molecule, said data being stored on the disc surface as numeric data, preferably readable by a CD or DVD reader.

60. The disc according to claim 43 wherein the capture molecules are specific molecules corresponding to data bytes.

61. The disc according to claim 60, wherein the data bytes capture molecules are present in an alignment on the disc surface.

62. The disc according to claim 60, wherein the alignment of capture molecules is converted into digital information, and form elements selected from the group consisting of words, numbers, music, software or data bases.

63. The disc according to claim 60, which comprises data bytes present on the same CD as both numeric informations and capture molecules.

64. A preparation method of the disc according to claim 43, which comprises the step of fixing upon the surface of a side of a disc opposite to the side comprising registered data, capture molecules through a photoactivation of an extremity of said capture molecules.

65. The method according to claim 64, wherein the fixing of the capture molecules is obtained through a covalent link between an extremity of the capture molecule and the surface layer of the disc.

66. The Method according to claim 64, wherein the disc surface is recovered by a protective layer, which allows or improves the protection and stabilisation of the capture molecule and/or the protection, stabilisation and/or detection of the binding between the target molecule and its corresponding capture molecule.

67. A diagnostic kit comprising the disc of the claim 43and reactants allowing the binding between a target molecule and its capture molecule and possibly reactants allowing the detection of a signal which results from said binding.

68. A detection and/or reading device which allows the detection and/or the quantification of a signal which results from the binding between a target molecule present in a sample and its capture molecule, and which comprises the disc according to any one of the claim 43 or the kit according to claim 67, and means for the detection and/or quantification of said signal.

69. The detection and/or reading device according to claim 68being a reading compact-disc device.
